# EUROPEAN PATENT APPLICATION

(11) **EP 4 302 675 A1**
(43) Date of publication of application: **10.01.2024**
(21) Application number: 22183657.0
(22) Date of filing: 07.07.2022
(51) Int. Cl.: A61B 1/00, A61B 1/005

(54) **BENDABLE TUBE FOR USE IN A SURGICAL DEVICE**

(71) Applicant: HOYA CORPORATION, Shinjuku-ku Tokyo 160-8347 (JP)
(72) Inventor: Rizzo, Marco, 86316 Friedberg (DE); Walberg, Erik, 86316 Friedberg (DE)
(74) Representative: Lucke, Andreas

(57) **Abstract**

Disclosed herein is a tube for use in a surgical device that is to be inserted into the human or animal body at least in part and a method for manufacturing such a tube. The tube according to the present disclosure extends from a proximal end to a distal end of the tube and includes a plurality of bendable segments. Each of the bendable segments includes three proximal openings, three central openings and three distal openings in a wall of the tube. The central openings are arranged along a circumference of the tube and separated from each other by central connecting portions of the wall. The central connecting portions are displaced from each other by between 105° and 135° along the circumference of the tube. The proximal openings are arranged on a proximal side of the central openings with each of the proximal openings being adjacent to a respective one of the central connecting portions. The distal openings are arranged on a distal side of the central openings with each of the distal openings being adjacent to a respective one of the central connecting portions.

## Description

### FIELD OF THE DISCLOSURE

The present disclosure is in the field of medical technology. In particular, the present disclosure relates to a bendable tube for use in a surgical device such as an endoscope that is to be inserted into the human or animal body at least in part and to a method of manufacturing such a tube.

### BACKGROUND

An endoscope is a surgical device that may be used to access (e.g., view or remove) or treat tissue within the body of a patient by inserting one or more medical tools into the body through an incision in the body or an orifice of the body. The endoscope may include (comprise) an interface/control portion and an insertion tube that is coupled to the interface/control portion. The insertion tube is configured to be inserted into the body of the patient and may include one or more channels to provide access to tissue within the body. The one or more channels may e.g. be configured to receive a medical tool and/or a fluid and to guide the medical tool and fluid, respectively, to the tissue of interest.

The insertion tube may be bendable to facilitate insertion into the patient's body. For this, the insertion tube may for example include one or more passively and/or actively bendable sections (also referred to as passive and active bending section, respectively). A passively bendable section may e.g. be formed of a bendable or flexible material or structure, whereas an actively bendable section may e.g. include one or more joints that can be articulated for actively bending the insertion tube.

While being bendable, the insertion tube must at the same time also exhibit sufficient rigidity, for example to allow a physician to push and rotate the insertion tube within the patient's body and/or to prevent plastic deformation when the insertion tube is bent. To achieve this, the insertion tube may for example be formed of a rigid or semi-rigid material and may be made bendable by providing a plurality of small openings in a wall of the insertion tube to reduce its stiffness, e.g. as disclosed in US 2021/0393111 A1. Yet, while openings in the wall of the insertion tube may provide flexibility, the openings may also reduce the torsional stiffness of the insertion tube. This may e.g. result in an insufficient transmission of torque or rotational movements along the tube. Furthermore, the openings may make the insertion tube prone to plastic deformation at small bending radii. Similar issues may also be encountered in actively bendable sections. Meeting these conflicting requirements may lead to complex structures that are difficult, time-consuming and thus costly to manufacture.

### SUMMARY OF THE DISCLOSURE

It is thus an object of the present disclosure to provide a tube for use in a surgical device such as an endoscope that is bendable while also providing sufficient torsional stiffness and resistance to plastic deformation.

This object is met by a tube for use in a surgical device according to claim 1 and a method of manufacturing a tube for use in a surgical device according to claim 14. Examples thereof are detailed in the dependent claims.

The tube according to the present disclosure is configured for use in a surgical device that is to be inserted into the human or animal body at least in part. The tube extends from a proximal end to a distal end of the tube and includes (i.e. including, but not limited to) a plurality of bendable segments. Each of the bendable segments includes three proximal openings, three central openings and three distal openings in a wall of the tube. The central openings are arranged along a circumference of the tube and are separated from each other by central connecting portions of the wall. The central connecting portions are displaced from each other by between 105° and 135° along the circumference of the tube. The three proximal openings are arranged on a proximal side of the central openings, wherein each of the proximal openings is adjacent to a respective one of the central connecting portions. The three distal openings are arranged on a distal side of the central openings, wherein each of the distal openings is adjacent to a respective one of the central connecting portions.

As used herein, the term "proximal" may refer to an element, a feature or a location along the length of the tube that is to be closer to a physician or other medical practitioner during use of the surgical device (e.g. closer to an interface/control portion of the surgical device), whereas the term "distal" may refer to an element, a feature or a location along the length of the tube that is to be closer to a location of tissue being examined or treated within the body of a patient during use of the surgical device (e.g. further away from an interface/control portion of the surgical device). A direction from the proximal end of the tube to the distal end may also be referred to as a longitudinal or axial direction in the following. A direction along a circumference of the tube (for example parallel to a surface, e.g. an outer surface, of the tube and perpendicular to the longitudinal direction) may also be referred to as a circumferential or azimuthal direction. A direction perpendicular to a surface, e.g. an outer surface, of the tube (for example perpendicular to the longitudinal direction and to the circumferential direction) may also be referred to as a radial direction. The longitudinal, circumferential and radial direction may for example define a cylindrical coordinate system. Depending on the state or configuration of the tube, the orientation of these directions may vary along the length of the tube, e.g. as a result of a bending of the tube.

The plurality of bendable segments may for example include between 2 and 1000 bendable segments, in some examples between 20 and 500 bendable segments. The bendable segments may be arranged along the length of the tube, e.g. in one or more sections of the tube as detailed below, for example in one or more passive bending sections arranged between the proximal end of the tube and an active bending section at the distal end of the tube.

Each of the proximal, central and distal openings may extend through the wall of the tube, e.g. from an inner surface of the wall facing the interior of the tube to an outer surface of the wall facing the exterior of the tube. In some examples, the tube may include one or more additional layers that are arranged on the inside and/or on the outside of the wall, e.g. a sleeve or cover surrounding the outer surface of the wall. Accordingly, the openings do not necessarily provide a fluid connection between the interior and the exterior of the tube, but may be covered on the inside and/or on the outside of the wall at least in part.

The central connecting portions are portions of the wall arranged between adjacent central openings, for example portions of the wall that remain when forming (e.g. cutting) the openings in the wall. The central connecting portions are displaced from each other by a displacement angle between 105° and 135°, in some examples by between 115° and 125°, in one example by between 118° and 122° (e.g. by 120°) along the circumference of the tube (along the circumferential direction). Accordingly, the central openings may also be displaced from each other by between 105° and 135°, in some examples by between 115° and 125°, in one example by between 118° and 122° (e.g. by 120°) along the circumference of the tube. The displacement angle may for example be the azimuthal angle enclosed by a triangle formed by the centers (e.g. centroids) of two adjacent central connecting portions (or central openings) and a center of the tube (e.g. a point along a central axis of the tube at the respective location along the length of the tube). Put differently, a distance between the centers of two adjacent central connecting portions (or central openings) may be between about 29.1% and 37.5%, in some examples between about 31.9% and about 34.7%, in one example between about 32.8% and about 33.9% (e.g. 1/3) of the circumference of the tube in the respective bendable segment.

The proximal openings are arranged on the proximal side of the central openings (e.g. between the central openings and the proximal end of the tube). The distal openings are arranged on the distal side of the central openings (e.g. between the central openings and the distal end of the tube). The proximal and distal openings are arranged adjacent to a respective one of the central connecting portions, e.g. such that each of the central connecting portions is arranged ("sandwiched") between a proximal opening and a distal opening. Some or all of the proximal and/or distal openings maybe aligned with the respective central connecting portion, for example such that a center of the proximal (or distal) opening is aligned with a center of the respective central connecting portion (e.g. located at the same or substantially the same azimuth).

At least some of the bendable segments (e.g. at least a quantity of bendable segments of the plurality of segments), in some examples all of the bendable segments, may be arranged in pairs along the longitudinal direction of the tube, wherein a distance between the bendable segments of each pair may be less than a distance between the respective pair and an adjacent pair. The distance between the bendable segments of each pair may be for example be less than 50%, preferably less than 25%, in one example less than 15% of the distance between the respective pair and an adjacent pair. The distance between bendable segments of a pair may for example be a distance between the distal openings of a proximal bendable segment of the respective pair and the proximal openings of a distal bendable segment of the respective pair. The distance between a pair and an adjacent pair may for example be a distance between the proximal openings of a proximal bendable segment of the pair and the distal openings of a distal bendable segment of the adjacent pair or a distance between the distal openings of a distal bendable segment of the pair and the proximal openings of a proximal bendable segment of the adjacent pair.

Additionally or alternatively, a distance between the distal openings of a proximal bendable segment and the proximal openings of a distal bendable segment of two adjacent bendable segments (e.g. for at least two adjacent bendable segments, in some examples for some or any two adjacent bendable segments) of the plurality of bendable segments may be between 50% and 200%, preferably between 80% and 120%, in one example between 90% and 110% of a distance between the proximal openings and the central openings of the distal bendable segment and/or between 50% and 200%, preferably between 80% and 120%, in one example between 90% and 110% of a distance between the central openings and the distal openings of the proximal bendable segment. In other words, the distance between adjacent bendable segments (e.g. between the proximal and distal bendable segments of a pair of segments) may be similar or comparable to the distance between the proximal and central openings and/or the distance between the central and distal openings of one or both of the adjacent bendable segments.

The proximal openings of the distal bendable segment and the distal openings of the proximal bendable segment of the two adjacent bendable segments may overlap along the circumferential direction of the tube, thereby forming spring segments therebetween (e.g. six spring segments arranged along the circumference of the tube with each of the spring segments extending between a respective proximal connecting portion of the distal bendable segment and a respective distal connecting portion of the proximal bendable segment). Each of the spring segments may be configured to be deformed (e.g. elongated and/or bent) when the tube is bent. A deformation of a spring segment may for example be associated with an opening (e.g. an increase of the cross-sectional area) or a closing (e.g. a decrease of the cross-sectional area) of the respective proximal and distal openings. Additionally or alternatively, each of the spring segments may be configured to provide a restoring force, wherein the restoring force may for example counter-act a bending or tilting of the respective bendable segments with respect to each other (e.g. drive the tube towards a straight/non-bent configuration). Providing spring segments between adjacent bendable segments may allow for increasing the flexibility of the tube.

A length of the spring segments along the circumferential direction may for example be between 5% and 16%, in some examples between 10% and 16%, in one example between 13% and 16% of the circumference of the tube between the respective bendable segments. Increasing a length of the spring segments may increase the flexibility of the tube. A width of the spring segments along the longitudinal direction may be uniform or approximately uniform along their length. In other examples, a width of one or more of the spring segments, in some examples of all of the spring segments at a center of the respective spring segment may be smaller than a width of the respective spring segment at one or both ends of the respective spring segment. The width at the center of the respective spring segment may for example be between 50% and 95%, in some examples between 60% and 90%, in one example between 65% and 80% of the width at one or both ends of the respective spring segment. The width of the spring segments may influence the flexibility of the tube and may be tuned to adjust the flexibility, e.g. to increase the flexibility by reducing the width of the spring segments. Increasing the width at the ends of a spring segment as compared to its center may provide improved guiding of torsional and/or bending forces into the respective spring segment.

In some examples, some or all of the bendable segments may be arranged as a contiguous group of bendable segments. A distance between the distal openings of a proximal bendable segment and the proximal openings of a distal bendable segment of any two adjacent bendable segments in the contiguous group of bendable segments maybe similar or comparable to a distance between the proximal openings and the central openings of the distal bendable segment and/or to a distance between the central openings and the distal openings of the proximal bendable segment. For example, the distance between the distal openings of a proximal bendable segment and the proximal openings of a distal bendable segment of any two adjacent bendable segments in the contiguous group of bendable segments may be between 50% and 200%, in some example between 80% and 120% of the distance between the proximal openings and the central openings of the distal bendable segment and/or between 50% and 200%, in some examples between 80% and 120% of the distance between the central openings and the distal openings of the proximal bendable segment. This may for example allow for providing spring segments both within and between the bendable segments.

Adjacent bendable segments of the plurality of bendable segments may be rotated with respect to each other along the circumferential direction, e.g. such that the central connecting portions of adjacent bendable segments are displaced from each other along the circumference of the tube. The central connecting portions of adjacent bendable segments may for example be displaced by between 52.5° and 67.5°, in some examples by between 57.5° and 62.5°, in one example by between 59° and 61° (e.g. by 60°) along the circumference of the tube. Adjacent bendable segments may in particular be rotated with respect to each other such that centers of the central openings of one of said adjacent bendable segments are aligned with the central connecting portions of another one of said adjacent segments, e.g. such that the center of the respective central opening is displaced from the respective central connecting portion by less than 5°, preferably by less than 2°, in one example by less than 1° and in one example by less than 0.5° along the circumference of the tube. Displacing adjacent bendable segments along the circumferential direction may lead to more homogeneous bending properties of the tube in some examples, e.g. by providing six primary bending directions/planes defined by centers of the openings rather than three primary bending directions/planes.

In some examples, the proximal, central and distal openings may all have the same size and/or shape, e.g. the same length along the circumferential direction and the same width along the longitudinal direction. In other examples, the proximal openings and/or the distal openings may have a different size and/or shape than the central openings, in particular a different width. For example, the proximal openings and/or the distal openings may be slits. As used herein, a "slit" may refer to an opening having a large aspect ratio (e.g. length to width), wherein the aspect ratio of a slit may for example be larger than 10:1, in some examples larger than 20:1, in one example larger than 50:1 and in one example larger than 100:1. A width of the central openings along the longitudinal direction may be at least 5 times, in some examples at least 10 times as large as a width of the proximal openings and/or the distal openings. In other examples, the central openings may be slits. In this case, the width of the proximal openings and/or of the distal openings along the longitudinal direction may for example be at least 5 times, in some examples at least 10 times as large as the width of the central openings.

The proximal openings maybe separated from each other by proximal connecting portions of the wall, for example portions of the wall remaining between the proximal openings when forming (e.g. cutting) the proximal openings. The distal openings maybe separated from each other by distal connecting portions of the wall, for example portions of the wall remaining between the distal openings when forming (e.g. cutting) the distal openings.

A length of the proximal connecting portions and/or a length of the distal connecting portions along the circumferential direction may be similar or comparable to a length of the central connecting portions, for example between 25% and 200%, in some examples between 30% and 100% of the length of the central connecting portions. In some examples, the length of the proximal connecting portions and/or the length of the distal connecting portions along the circumferential direction may be smaller than the length of the central connecting portions, for example between 30% and 95%, in some examples between 50% and 80% of the length of the central connecting portions. The length of the proximal connecting portions and/or the length of the distal connecting portions may be small compared to the circumference of the tube in the respective bendable segment, for example no more than 10%, in some examples no more than 5% of the circumference of the tube in the respective bendable segment. Additionally or alternatively, the length of the central connecting portions may be small compared to the circumference of the tube in the respective bendable segment, for example no more than 15%, in some examples no more than 10%, in one example no more than 8% of the circumference of the tube in the respective bendable segment. Reducing a length of the proximal, central and/or distal connecting portions may allow for increasing a length of spring segments adjacent to the respective connecting portions and may lead to an increased flexibility of the tube. Increasing a length of the proximal, central and/or distal connecting portions may provide increased torsional stiffness of the tube.

Some or all of the bendable segments (e.g. at least a quantity of the bendable segments of the plurality of segments) each may further include one or more additional sets of openings in addition to the proximal, central and distal openings. For example, some or all of the bendable segments may include a fourth set of openings, wherein the fourth set of openings may either be arranged on a proximal side of the proximal openings or on a distal side of the distal openings. The fourth set of openings may include three fourth openings, wherein each of the fourth openings may e.g. be adjacent to a respective one of the proximal and distal connecting portions, respectively. In one example, the two inner sets of openings (e.g. the central openings and the proximal openings with the fourth openings being arranged on the proximal side of the proximal openings) maybe slits. A width of the openings of the two outermost sets of openings (i.e. the distal openings and the fourth openings in the aforementioned example) along the longitudinal direction may be at least 5 times, in some examples at least 10 times as large as a width of the openings in the two inner sets of openings. Bendable segments with four sets of openings may for example provide improved torque transfer at a given rigidity/flexibility of the tube.

The proximal openings and/or the distal openings of some or all of the bendable segments (e.g. of at least a quantity of bendable segments of the plurality of segments) may overlap with the central openings of the respective bendable segment along the circumferential direction, thereby forming spring segments therebetween. For example, all of the proximal openings may each overlap with the two central openings separated by the respective proximal connecting portion. Thereby, a set of six spring segments may be formed between the proximal and central openings with the six spring segments being arranged along the circumference of the tube. Additionally or alternatively, a set of six spring segments may be formed between the central and distal openings in this way. The spring segments may for example be similar as described above for the spring segments between adjacent bendable segments, in particular with regard to their length and/or width. In some examples, a width of one or more of said spring segments along the longitudinal direction at a center of the respective spring segment is smaller than a width of the respective spring segment at one or both ends of the respective spring segment, e.g. as described above for the spring segments between adjacent bendable segments. The length, width and shape of the spring segments may be tuned to adjust the flexibility of the tube as described above for the spring segments between adjacent bendable segments.

A width along the longitudinal direction of some or all of the central openings may be uniform or approximately uniform along their length. The central openings may for example have a rectangular shape, wherein corners of the rectangle may be rounded. Additionally or alternatively, some or all of the central openings (e.g. two or more of the central openings) each may include a constricted central portion at a center of the respective central opening. The constricted central portion may have a width along the longitudinal direction that is smaller than a width of adjacent portions of the central opening on both sides of the central portion. The constricted central portion may have a width along the longitudinal direction that is smaller than a maximum width of the respective central opening, for example between 20% and 90%, in some examples between 40% and 70% of a maximum width of the respective central opening. The constricted central portion be configured to limit a compression of the respective central opening in the longitudinal direction, e.g. due to sidewalls of the central opening coming in contact with each other. The constricted central portion may for example be configured to limit the compression of the respective central opening in the longitudinal direction to the width of the respective central portion.

Additionally or alternatively, a width of some or all of the central openings (e.g. of two or more of the central openings) along the longitudinal direction at the center of the respective central opening may be smaller than a width of one or both end portions of the respective central opening, between 20% and 90%, in some examples between 40% and 70% of the width of one or both end portions. In other examples, the width of one or both end portion of the respective central opening may be larger than the width at the center, e.g. as described below. The width of the end portions of a central opening may for example be measured at 5% and 95%, respectively, in some examples at 10% and 90%, respectively, of the length of the respective central opening or may be defined as a maximum width of the end portions, wherein the end portions may for example include the outermost 5%, in some examples the outermost 10% of the length of the respective central opening. In some examples, the end portions may include no more than the outermost 5%, in one example no more than the outermost 10% of the length of the respective central opening. In some examples, the end portions may each include a rounded corner portion, wherein the width of the end portions may e.g. be measured at an innermost edge of the rounded corner portions.

Some or all of the central openings (e.g. two or more of the central openings) each may include widened intermediate portions arranged between the central portion and a respective end portion of the respective central opening. A width of the widened intermediate portions along the longitudinal direction may be larger than the width of the central portion and larger than a width of the end portions. For example, the width of the central portion and/or the width of the end portions may be between 20% and 90%, in some examples between 40% and 70% of the width of the intermediate portions. The width of the widened intermediate portions may constitute the maximum width of the respective opening. In some examples, the width of one or both end portions of the respective central opening may be smaller than the width of the central portion, for example between 40% and 90%, in one example between 40% and 70% of the width of the central portion.

In some examples, some or all of the proximal openings (e.g. two or more of the proximal openings) and/or some or all of the distal openings (e.g. two or more of the distal openings) may be shaped as described for the central openings above, in particular in examples where the central openings are slit-shaped, but also in other examples where the central openings e.g. include a constricted central portion and/or widened intermediate portions. One or both of the proximal and distal openings may for example include a constricted central portion and/or widened intermediate portions.

In some examples, the bendable segments maybe arranged in two or more sections, in some examples in three or more sections, in one example in four or more sections and in one example in five or more sections along the length of the tube. The sections maybe adjacent to each other or may be spaced apart, e.g. such that a distance between the sections is substantially larger than the distance between bendable segments within the sections. In some examples, the sections may differ in a density of bendable segments, wherein the density may for example characterize a number of bendable segments per unit length of the tube and/or an inverse of a distance between adjacent bendable segments or pairs of bendable segments in the respective section. For example, a density of bendable segments in each of the two or more sections maybe different from a density of bendable segments in at least one other section, in some examples in all other sections. The density of bendable segments/distance between adjacent bendable segments or pairs may be used as a parameter for tuning the stiffness of the respective section.

The tube may for example include a first section and a second section (the first and second sections being included in said two or more sections), wherein a density of bendable segments in the first section is different from a density of bendable segments in the second section. The density of bendable segments in the second section may for example be at least 5%, in some examples at least 10%, in one example at least 20% larger than in the first section, e.g. to provide sections with slightly different stiffness. In other examples, the density of bendable segments in the second section may be at least 50%, in some examples at least 100%, in one example at least 150% larger than in the first section, e.g. to provide sections with substantially different stiffness.

Additionally or alternatively, the two or more sections may differ in the way how the bendable segments are arranged. For example, the bendable segments in at least one of said two or more section, e.g. in the second section, maybe arranged as a contiguous group of bendable segments with a distance between the distal openings of a proximal bendable segment and the proximal openings of a distal bendable segment of any two adjacent bendable segments in the contiguous group of bendable segments being similar or comparable to a distance between the proximal openings and the central openings of the distal bendable segment and/or to a distance between the central openings and the distal openings of the proximal bendable segment. For example, the distance between the distal openings of the proximal bendable segment and the proximal openings of the distal bendable segment may be between 50% and 200%, in some examples between 80% and 120% of the distance between the proximal openings and the central openings of the distal bendable segment and/or between 50% and 200%, in some example between 80% and 120% of the distance between the central openings and the distal openings of the proximal bendable segment. This may for example allow for providing spring segments both within and between the bendable segments.

The bendable segments in at least one of said two or more sections, e.g. in the first section, may for example be arranged in pairs along the longitudinal direction, e.g. with the distance between the bendable segments of each pair being less than 50%, in some examples less than 25%, in one example less than 15% of the distance between the respective pair and an adjacent pair. In some examples, the bendable segments in one or more other sections of said two or more sections, for example in the second section, may also be arranged in pairs, but e.g. at a different distance between adjacent pairs. In other examples, the bendable segments in both the first section and the second section maybe arranged as a respective contiguous group of bendable segments as described above, but e.g. at different distances between adjacent bendable segments. The distance between adjacent pairs may for example be used as a parameter for tuning the density and thus the stiffness of the respective section.

The two or more sections may include one or more, in some examples two or more additional sections in addition to the first and second sections. In other words, the tube may include three or more sections, said three or more sections including the first and second sections. A density of bendable segments in each of said additional sections maybe different from a density of bendable segments in at least one other section, in some examples all other sections of said two or more section, e.g. different from the density of bendable segments in the first section and/or in the second section. In some examples, the bendable segments in some or all of the additional sections may also be arranged in pairs as described above and/or in respective contiguous groups as described above.

In addition to or instead of the density of bendable segments and/or the arrangement of bendable segments, the two or more sections may also differ in one or more parameters pertaining to the arrangement and/or shape of the openings in the bendable segments and/or in the pairs of bendable segments. For example, each of said two or more sections may differ in one or more of these parameters from at least one other section, in some examples from every other section. The bendable segments in the first section may for example differ from the bendable segments in the second section and/or from the bendable segments in some or all of the one or more additional sections in one or more of: (1) the distance between the proximal openings and the central openings and/or the distance between the central openings and the distal openings, e.g. in the width of the spring segments formed between the proximal and central openings and/or the central and distal openings; (2) the distance between bendable segments within a pair of bendable segments, e.g. in the width of the spring segments formed between the bendable segments of the pair; (3) the length of the proximal connecting portions between the proximal openings and/or the length of the distal connecting portions between the distal openings; and (4) the length of the central connecting portions between the central openings. Additionally or alternatively, the sections may also differ in other parameters pertaining to the arrangement and/or shape of the openings, e.g. in the width of one or more of the constricted central portions, the intermediate portions and the end portions of the central openings.

In some examples, the tube may further include an active bending section with a plurality of annular or tubular elements that are pivotably connected to each other by a plurality of joints. Each of the joints may for example pivotably connect a respective proximal element to a respective distal element. The joints may for example be hinges that allow for articulation or rotation about a single axis of rotation. In some examples, adjacent elements of the active bending section may be connected by a pair of joints arranged on opposite sides of the tube, e.g. rotated with respect to each other by 180° along the circumferential direction. The tube may also include means for actuating the joints, for example one or more control wires as commonly known in the art. The active bending section may for example be arranged between the plurality of bendable segments and the distal end of the tube, e.g. at or adjacent to the distal end of the tube.

In some examples, some or all of the joints each include a distal contact surface on a proximal element associated with the respective joint that is configured to slidably engage a proximal contact surface on a distal element associated with the respective joint. A reference protrusion may be arranged on one (e.g. a first one) of the proximal contact surface and the distal contact surface. Additionally or alternatively, a reference notch may be arranged on the other one (e.g. a second one) of the proximal contact surface and the distal contact surface. In other words, at least one of a reference protrusion and a reference notch is/are arranged on at least one of the proximal and distal contact surfaces. The at least one of the reference protrusion and the reference notch (i.e. the reference protrusion and/or the reference notch) may be arranged such that a force that is required for pivoting the proximal and distal elements with respect to each other is higher when the joint is in a reference position (e.g. a first articulation angle) than when the joint is in a second position (e.g. a second articulation angle) different from the reference position. This may for example allow for biasing the joint towards the reference position, e.g. such that the joint is preferably positioned in the reference position when no tension is applied to the active bending section.

The at least one of the reference protrusion and the reference notch (i.e. the reference protrusion and/or the reference notch) may for example be arranged such that less friction is generated within the joint in the reference position than in the second position. For example, an overlap or a contact area between the proximal and distal contact surfaces (e.g. a surface area of the proximal contact surface that is in contact with the distal contact surface) increases when the joint is moved from the reference position to the second position.

Additionally or alternatively, the at least one of the reference protrusion and the reference notch (i.e. the reference protrusion and/or the reference notch) may be arranged such that a length of the tube increases when the joint is moved from the reference position to the second position. In one example, a reference protrusion is arranged on one of the proximal and distal contact surfaces and a reference notch is arranged on the other one of the proximal and distal contact surfaces. In the reference position, the reference protrusion may be arranged in the reference notch at least in part. In the second position, the reference protrusion may be arranged outside of the reference notch. This may lead to an increase in the length of the tube when the joint is moved from the reference position to the second position. Pivoting the proximal and distal elements with respect to each other in the reference position may thus require a larger force than in the second position since the reference protrusion has to be moved out of the reference notch.

In some examples, the proximal element and the distal element are parallel to each other when the joint is in the reference position, e.g. such that an articulation angle between the proximal and distal elements is zero or substantially zero. Outer surfaces of the proximal and distal elements may for example be flush and parallel to each other in the reference position (e.g. such that the corresponding portion of the tube is straight or substantially straight). In other examples, the proximal and distal elements maybe arranged at a non-zero articulation angle in the reference position, e.g. such that the corresponding portion of the tube is bent with a pre-defined bending radius.

Some or all of the joints may each include a pair of arc-shaped proximal brackets on a distal element associated with the respective joint, wherein each of the proximal brackets extends along a circular arc. Additionally or alternatively, some or all of the joints may each include a pair of arc-shape distal brackets on a proximal element associated with the respective joint, wherein each of the distal brackets extends along a respective circular arc. The proximal brackets and/or the distal brackets may for example extend along a circular arc having a central angle of at least 30°, in some examples of at least 60°, in one example of at least 90°, in one example of at least 120° and/or of no more than 170°, in some examples of no more than 150°, in one example of no more than 120°.

Each of the proximal brackets may be configured to slidably engage with a corresponding recess in the proximal element and/or with one of the distal brackets. Additionally or alternatively, each of the distal brackets may be configured to slidably engage with a corresponding recess in the distal element and/or with one of the proximal brackets. The proximal brackets and/or the distal brackets may for example be configured to slidably engage with the corresponding recess such that one of the respective brackets is moved further into the recess when the joint is articulated in a first direction, whereas the other one of the respective brackets is moved further out of the recess when the joint is articulated in the first direction, and vice-versa. Similarly, the proximal brackets and/or the distal brackets may for example be configured to slidably engage with the corresponding brackets ("counter-brackets") such that an overlap and/or a contact area between one of the respective brackets and the corresponding counter-bracket increases when the joint is articulated in the first direction, whereas an overlap and/or a contact area between the other one of the respective brackets and the corresponding counter-bracket decreases when the joint is articulated in the first direction, and vice-versa. Providing the proximal and/or distal brackets may increase the robustness of the joint in some examples, e.g. such that the joint may be configured to transfer and/or resist larger bending and/or torsional forces.

The proximal brackets or the distal brackets may form a circular joint socket (e.g. a circular or substantially circular recess or cut-out) configured to receive a corresponding joint head (e.g. a circular or substantially circular protrusion or pin) on the distal element and the proximal element, respectively. In some examples, the reference protrusion may be arranged on a surface of one (e.g. a first one) of the joint socket and the joint head. Additionally or alternatively, the reference notch may be arranged on a surface of the other one (e.g. a second one) of the joint socket and the joint head. In other words, at least one of the reference protrusion and the reference notch is/are arranged on surfaces of at least one of the joint socket and the joint head. For example, the reference protrusion may be arranged on a surface of the joint head and the reference notch maybe arranged on a surface of the joint socket or vice-versa.

The active bending section according to any one of the examples described above may also be used without the bendable segments according to the present disclosure, e.g. in a tube having different bendable segments or also in a tube having no passive bending sections and/or no bendable segments. Accordingly, the present disclosure also provides a tube for use in a surgical device that is to be inserted into the human or animal body at least in part, wherein the tube includes an active bending section with a plurality of annular or tubular elements that are pivotably connected to each other by a plurality of joints, wherein some or all of the joints each include a distal contact surface on a proximal element that is configured to slidably engage a proximal contact surface on a distal element, wherein a reference protrusion and/or a reference notch is/are arranged on the proximal contact surface and/or on the distal contact surface such that a force that is required for pivoting the proximal and distal elements with respect to each other is higher when the joint is in a reference position than when the joint is in a second position different from the reference position. The joints in the active bending section may for example be embodied as described above. Said tube may further include any of the features of the tube according to any of the examples of the present disclosure described herein.

The tube according to the present disclosure may for example be a tube configured for use in an endoscope, for example and without limitation, a bronchoscope, a sinuscope, a naso-pharyngoscope, a laryngoscope, a laparoscope, a gastroscope, a duodenoscope, a colonoscope, an echoendoscope, a hysteroscope, a cystoscope, a uroscope, a urethroscope, a cardioscope, and an arthroscope. A length and/or a diameter of the tube may be chosen accordingly. The tube may for example be an insertion tube for use in an endoscope or a part of an insertion tube for use in an endoscope. In one example, the tube is a hypotube for use in an endoscope. The hypotube may for example be configured to be surrounded or enclosed by a tubular sleeve or cover to form an insertion tube, wherein the sleeve or cover may cover the openings of the bendable segments. The hypotube may for example form a frame or skeleton of the insertion tube that is configured to provide dimensional stability to the insertion tube. In addition or instead of endoscopes, the tube may also be configured for use in other surgical devices that are to be inserted into the human or animal body at least in part such as for example a catheter or a part thereof. The tube according to the present disclosure may for example be a tube configured for use in a guiding instrument to guide a treatment tool or light guide into the patient's body, e.g. during a laparoscopic surgery.

The present disclosure further provides a surgical device that is to be inserted into the human or animal body at least in part, in particular an endoscope, including a tube according to any one of the examples described herein. The tube may for example be an insertion tube of the endoscope or a part thereof, in particular a hypotube. The endoscope may further include an interface/control portion that is coupled to the tube.

The present disclosure further provides a method of manufacturing a tube for use in a surgical device that is to be inserted into the human or animal body at least in part. The method includes providing a tubular piece and forming a plurality of bendable segments in the tubular piece. Each of the bendable segments is formed by: (1) forming three central openings in a wall of the tubular piece, the central openings being displaced from each other along a circumference of the tubular piece by between 29% and 38% of the circumference of the tubular piece and separated from each other by central connecting portions of the wall; (2) forming three first openings in the wall on a first side of the central openings with each of the first openings being formed adjacent to a respective one of the central connecting portions; and (3) forming three second openings in the wall on a second side of the central openings opposite to the first side, wherein each of the second openings is formed adjacent to a respective one of the central connecting portions. The above numbering is for clarity only and does not imply a certain order of execution of the method. As far as technically feasible, the method may be executed in an arbitrary order and parts thereof may also be executed simultaneously at least in part.

The method may for example be used to manufacture a tube according to any one of the examples described herein, e.g. to form the bendable segments and the openings in the arrangement and shapes described herein. The first openings may for example be formed on a proximal side of the central openings and may correspond to the proximal openings of the tube according to the present disclosure. The second openings may for example be formed on a distal side of the central openings and may correspond to the distal openings of the tube according to the present disclosure. The method may further include forming additional openings in the tubular piece, e.g. a fourth set of openings as described above. The method may also include forming one or more active bending sections as described herein, e.g. in the tubular piece or as a separate piece that is connected or attached to the tubular piece. The one or more active bending sections may for example be formed using any one of the techniques for forming the bendable segments described herein.

The tubular piece may include (i.e. including, but not limited to) a rigid or semi-rigid material, in particular a metal such as stainless steel, titanium or a nickel-titanium alloy such as Nitionol. In some examples, the tubular piece may consist of (i.e. include nothing else but) a rigid or semi-rigid material, in particular a metal such as stainless steel, titanium or a nickel-titanium alloy such as Nitionol. Additionally or alternatively, the tubular piece may also include plastic. In some examples, the tubular piece may consist of plastic.

The openings may for example be formed by cutting the wall of the tubular piece, in particular by laser cutting. However, the method is not limited to a particular machining process or technique for forming the openings and the openings may also be formed by other means, for example using any suitable computer numerical control (CNC) machining technique such as drilling and/or milling.

The central openings are formed displaced from each other along the circumference of the tubular piece by between 29% and 38% (e.g. corresponding to a displacement angle between about 105° and about 135° relative to a center of a tube), in some examples between 31% and 35%, in one example between 32.8% and 33.9% (e.g. 1/3) of the circumference of the tubular piece in the respective bendable segment. The displacement of central openings may for example be measured between the centers (e.g. centroids) of the respective central openings. The central openings may in particular be formed such that the central connecting portions between the central openings are displaced as described above for the tube according to the present disclosure.

In some examples, the first openings and/or the second openings of some or all of the bendable segments are slits formed by laser cutting. Put differently, the method may include laser cutting slits as the first openings and the second openings. Additionally or alternatively, the central openings of some or all of the bendable segments may be slits formed by laser cutting. A width of the respective openings/slits along a longitudinal direction of the tubular piece may correspond to a cutting width of the laser cutting. The cutting width may for example be between 10 µm and 50 µm, in some examples between 20 µm and 40 µm.

By providing three central openings arranged along the circumference of the tube, the present disclosure allows for forming tubes for use in surgical devices that are bendable while at the same time exhibiting a sufficient degree of torsional stiffness and resistance to plastic deformation. The three central connecting portions of the tube wall separating the central openings are arranged with a three-fold or approximately three-fold symmetry about a center or axis of the tube and provide three approximately equidistant points for the direct transmission of forces across the respective bendable segment. Thereby, torque may be transmitted along the length of the tube efficiently, e.g. from the proximal to the distal end, while reducing torsional and/or bending stress in the bendable segments. Between the central openings and the proximal and distal openings, respectively, spring segments may be formed that connect the central connecting portions with the proximal/distal connecting portions. When the tube is bent, the spring segments may be deformed and may absorb stress, thus providing relief to other load points.

The bendable segments according to the present disclosure may reduce an influence of the inter-segment distances on the torsional stiffness of the tube and may thus allow for providing sufficient flexibility by adding bendable segments/reducing the inter-segment distances without significantly affecting torsional stiffness. Furthermore, the proposed design provides a plurality of parameter pertaining to the size, shape and arrangement of the openings that may be tuned to control mechanical properties of the tube as desired for the respective surgical device or intended application as detailed above. The bendable segments according to the present disclosure may be easy to manufacture and may allow for achieving a good compromise between processing time/cost, flexibility as well as torsional stiffness of the tube that may be adjusted flexibly depending on the respective requirements. For example, the number of bendable segments may be reduced and/or a shape of the openings simplified (e.g. by increasing radii of curvature associated with the openings) to allow for faster processing during manufacture.

### LIST OF FIGURES

In the following, a detailed description of the present disclosure and examples thereof is given with reference to the figures. The figures show schematic illustrations of
Fig. 1: an endoscope according to an example of the present disclosure;
Fig. 2a: a tube for use in a surgical device in accordance with an example of the present disclosure;
Fig. 2b: a bendable segment of the tube of Fig. 2a according to an example of the present disclosure;
Fig. 3a: a bendable segment of a tube for use in a surgical device including an opening with a constricted central portion according to another example of the present disclosure;
Fig. 3b: a pairwise arrangement of bendable segments as in Fig. 3a on a tube for use in a surgical device in accordance with an example of the present disclosure;
Fig. 4: a perspective view of a tube for use in a surgical device with spaced-apart bendable segments according to an example of the present disclosure;
Fig. 5a: a pairwise arrangement of bendable segments of a tube for use in a surgical device with constricted central portions in accordance with another example of the present disclosure;
Fig. 5b: a pairwise arrangement of bendable segments of a tube for use in a surgical device with widened intermediate portions in accordance with an example of the present disclosure;
Fig. 6a: a tube for use in a surgical device with bendable segments having central slits in accordance with an example of the present disclosure;
Fig. 6b: a tube for use in a surgical device with bendable segments having four sets of openings according to an example of the present disclosure;
Fig. 7: a tube for use in a surgical device including a plurality of passive bending sections with bendable segments as well as an active bending section in accordance with an example of the present disclosure;
Fig. 8a: bendable segments in a distal passive bending section of the tube of Fig. 7;
Fig. 8b: bendable segments in a central passive bending section of the tube of Fig. 7;
Fig. 8c: bendable segments in a proximal passive bending section of the tube of Fig. 7;
Figs. 9a, 9b: tube elements and joints in the active bending section of the tube of Fig. 7;
Fig. 9c: a joint interface of a joint in the active bending section of the tube of Fig. 7; and
Fig. 10: a flow chart of a method of manufacturing a tube for use in a surgical device according to an example of the present disclosure.

### DESCRIPTION OF EXAMPLES

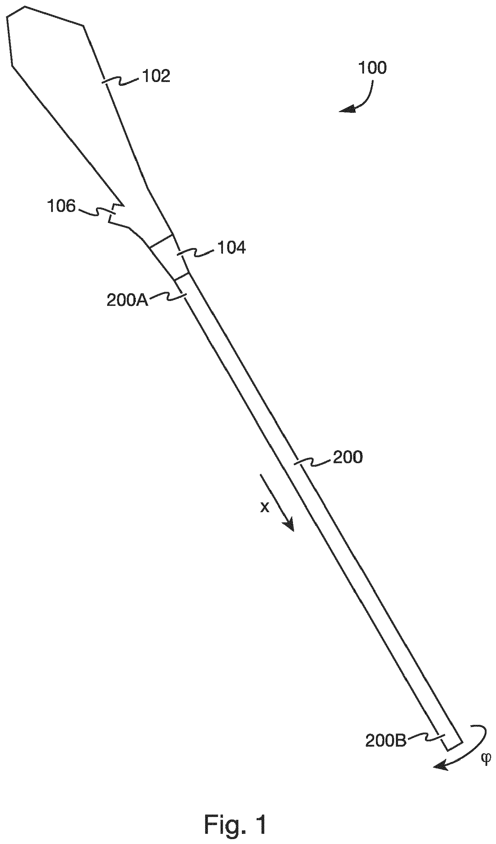

Fig. 1 shows a schematic illustration (not to scale) of a surgical device 100 according to an example of the present disclosure. In the example of Fig. 1, the surgical device 100 is an endoscope including an interface/control portion 102 with a connector 104 and one or more ports 106.

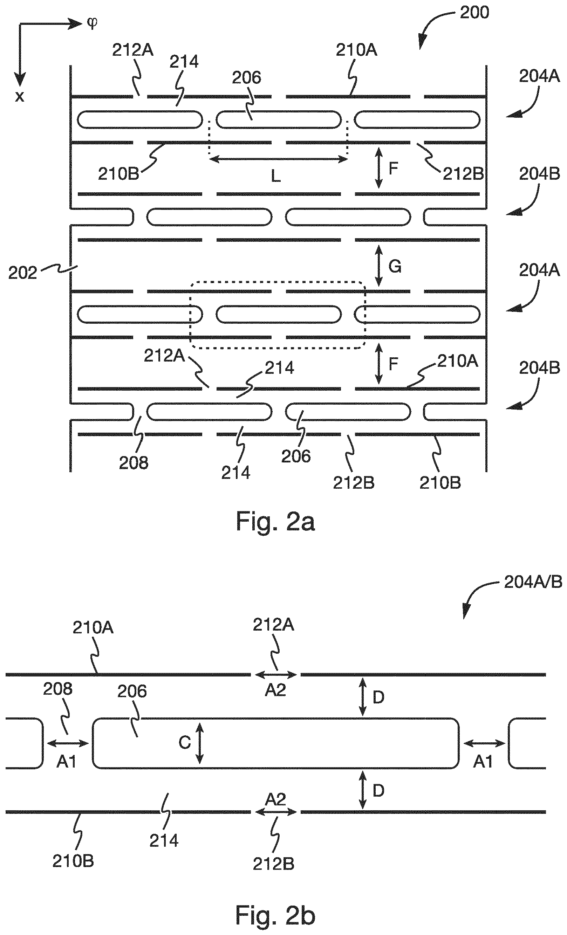

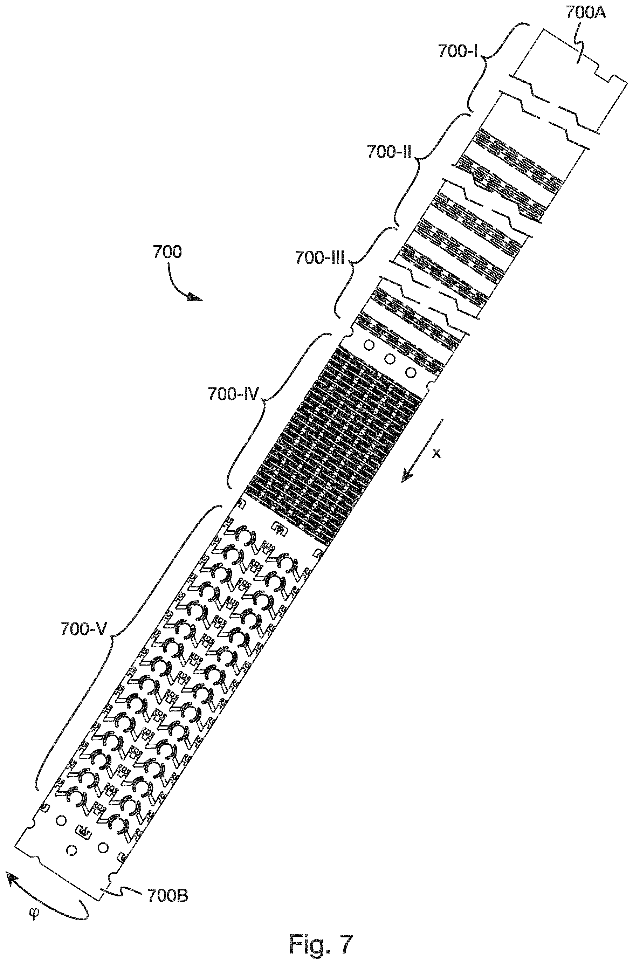

The connector 104 is configured to connect or attach a tube 200 to the interface/control portion 102, wherein the tube 200 may be connected or attached removably in some examples. The tube 200 may be a tube according to any one of the examples described herein, for example the tube 200 of Fig. 2a or the tube 700 of Fig. 7. The tube 200 has a proximal end 200A and a distal end 200B and extends from the proximal end 200A to the distal end 200B, wherein the proximal end 200A is adjacent to the interface/control portion 102 (e.g. connected or attached thereto via the connector 104) and the distal end 200B is facing away from the interface/control portion 102. A direction X from the proximal end 200A to the distal end 200B may be referred to as a longitudinal, axial or X direction in the following. A direction ϕ along a circumference of the tube (e.g. parallel to a surface of the tube and perpendicular to the longitudinal direction X) may be referred to as a circumferential, azimuthal or ϕ direction in the following.

The one or more ports 106 may for example include one or more fluid ports that are in fluid communication with an interior of the tube 200, for example with a channel (not shown) formed by or arranged in the tube 200, e.g. via the connector 104. Additionally or alternatively, the one or more ports 106 may include one or more light guide ports, wherein each of the light guide ports may for example be configured to receive a light guide (not shown) that is to be arranged within the tube 200 and/or to provide coupling to a light guide (not shown) that is arranged within the tube 200, for example in a channel formed by or arranged in the tube 200. The interface/control portion 102 may further include one or more electrical contacts (not shown), e.g. to provide an electrical connection to the interior of the tube 200, to a distal end 200B of the tube 200 and/or to a distal tip (not shown) of the endoscope 100, which may e.g. be arranged at or connected to the distal end 200B of the tube 200. The interface/control portion 106 may also include means for actuating an active bending section (not shown) of the tube 200 such as the active bending section 700-V of Fig. 7, for example one or more control knobs (not shown), which may e.g. be configured to apply tension to one or more control wires (not shown) coupled to the active bending section.

The interface/control portion 102 may be embodied as a single unit as shown in Fig. 1 or as two or more separate units, for example a control portion and an interface portion, wherein the control portion may e.g. be connected to the proximal end 200A of the tube 200 via the connector 104 and the interface portion may e.g. be connected to the control portion via a flexible tube or an umbilical cord.

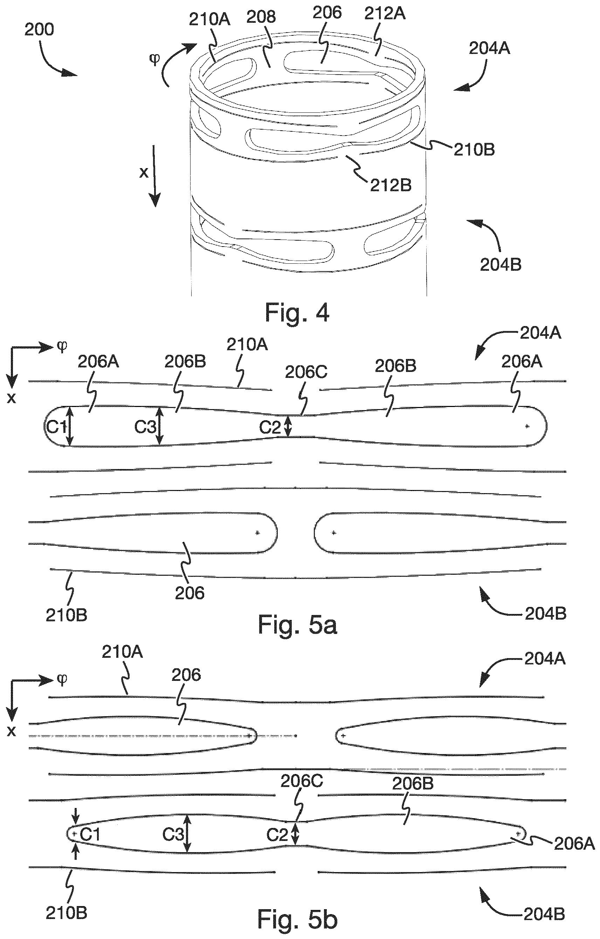

Fig. 2a depicts a schematic illustration (not to scale) of a tube 200 for use in a surgical device in accordance with an example of the present disclosure. The tube 200 may for example be configured for use in the endoscope 100 of Fig. 1. In Fig. 2a, the tube 200 is shown cut open along the longitudinal X direction with its wall 202 rolled out flat such that the entire circumference (360° along the circumferential direction) of the tube 200 is visible. Normally, e.g. during use, the wall 202 would be rolled up along the circumferential direction such that the left edge of the wall 202 in Fig. 2a is connected to the right edge of the wall 202, thereby forming a tubular structure, e.g. similar to the tube in Fig. 4 described below.

The tube 200 may for example have an elliptical cross-section, in particular a circular cross-section, e.g. such that the tube 200 as a cylindrical shape. A length and a diameter of the tube 200 may be chosen to be suitable for the type of endoscope(s) that the tube 200 is to be used with. The tube 200 may for example have a length between 20 cm and 200 cm, in some examples between 50 cm and 150 cm. An outer diameter of the tube 200 may for example be between 1 mm and 20 mm, in some examples between 2 mm and 10 mm. The tube 200 may include (i.e. including, but not limited to) a rigid or semi-rigid material, in particular a metal such as stainless steel. In one example, the tube 200 may consists of (i.e. including nothing else but) a rigid or semi-rigid material, in particular a metal such as stainless steel. The tube 200 may for example be a hypotube for the endoscope 100. In other examples, the tube 200 may be an insertion tube for the endoscope 100. The insertion tube may for example further include a sleeve or cover (not shown) surrounding a surface of the wall 202, e.g. an inner surface and/or an outer surface of the wall 202 (or put differently, the wall 202 may form a hypotube of the insertion tube in some examples), wherein the sleeve or cover may include of a flexible material such as plastic or rubber. In one example, the sleeve or cover may consist of a flexible material such as plastic or rubber.

The tube 200 includes a plurality of bendable segments 204A, 204B, wherein each of the bendable segments 204A, 204B includes three sets of openings in the wall 202 of the tube 200: three central openings 206, three proximal openings 210A on a proximal side of the central openings 206 (i.e. between the central openings 206 and the proximal end 200A) and three distal openings 210B on a distal side of the central openings 206 (i.e. between the central openings 206 and the distal end 200B). The openings in each set (proximal, central and distal) are arranged along a circumference of the tube 200 and are separated from each other by proximal connecting portions 212A, central connecting portions 208 and distal connecting portions 212B, respectively, of the wall 202. The proximal, central and distal openings 206, 210A, 210B may be perpendicular to the longitudinal direction, i.e. may extend parallel to the circumferential direction.

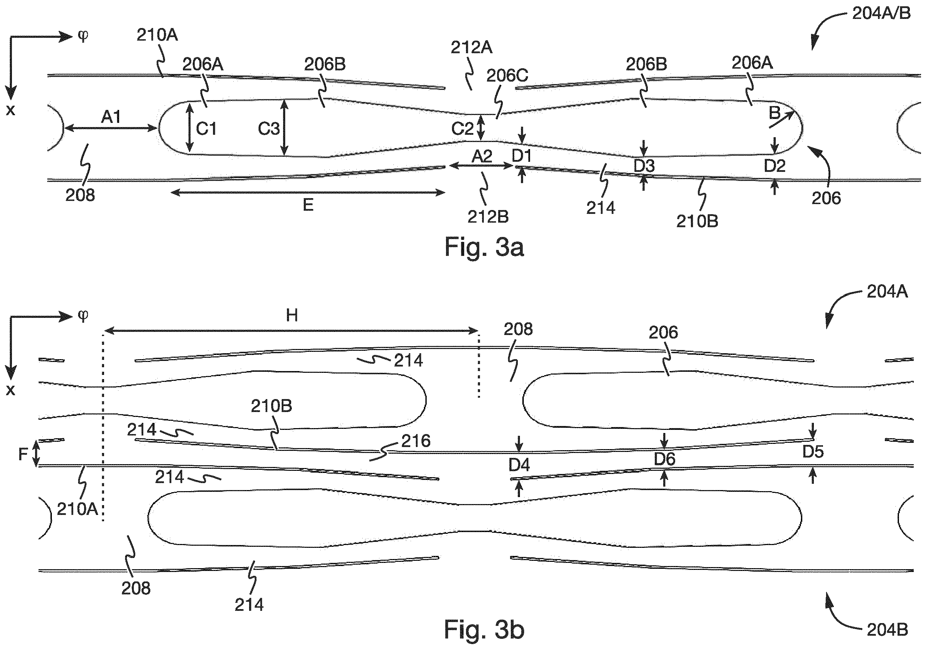

In the example of Fig. 2a, the connecting portions associated with each set are displaced from each other along the circumference of the tube 200 by a displacement angle of 120°, for example such that a length/displacement L between the centers of two adjacent central connecting portions is equal to 1/3 of the circumference of the tube 200. In other examples, the displacement angle may deviate slightly from 120° and may for example be between 105° and 135°, in some examples between 118° and 122°. The sets of proximal and distal openings 210A, 210B are rotated (or displaced) with respect to the set of central openings 206 by half the displacement angle (corresponding to a shift/displacement by H = L/2 along the circumferential direction, cf. Fig. 3b), e.g. by 60°, such that each of the proximal and distal openings 210A, 210B is adjacent to a respective one of the central connecting portions 208. In the example of Fig. 1, the center of each of the proximal and distal openings 210A, 210B is aligned with a center of the respective central connecting portions 208 along the circumferential direction.

In the example of Fig. 2a, the plurality of bendable segments 204A, 204B includes two sets of bendable segments, namely a first set of bendable segments 204A and a second set of bendable segments 204B. The bendable segments 204B in the second set are rotated (or displaced) by half the displacement angle with respect to the bendable segments 204A in the first set, e.g. by 60°. Accordingly, centers of the central openings 206 of the bendable segments 204B in the second set are aligned with the central connecting portions 208 of the bendable segments 204A in the first set and vice-versa. Centers of the proximal openings 210A of the bendable segments 204B in the second set are aligned with the distal connecting portions 212B of the bendable segments 204A in the first set and vice-versa. Centers of the distal openings 210B of the bendable segments 204B in the second set are aligned with the proximal connecting portions 212A of the bendable segments 204A in the first set and vice-versa. Apart from being rotated, the bendable segments 204A, 204B may be identical in some examples.

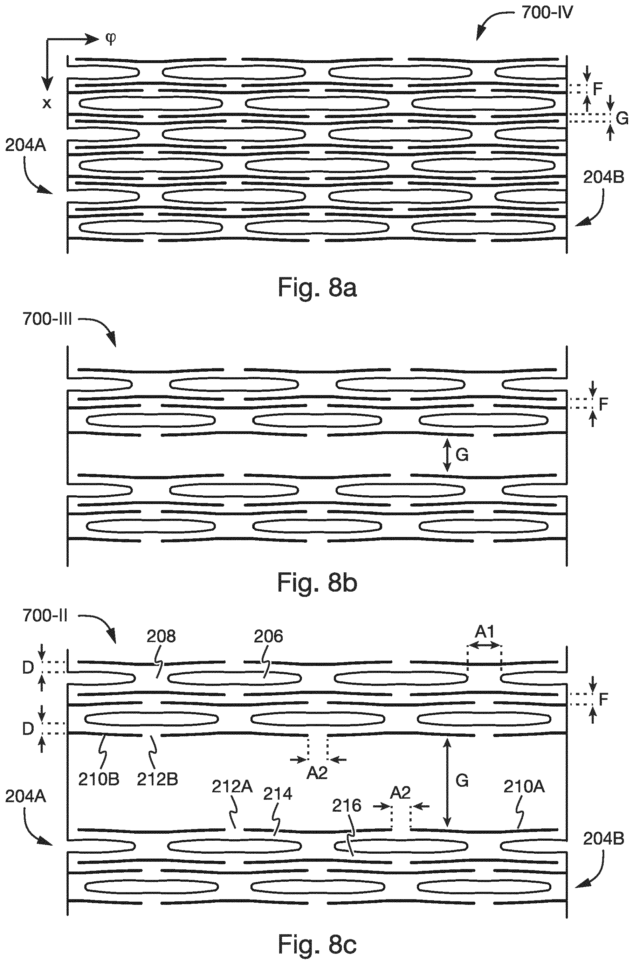

The bendable segments 204A, 204B in the two sets are arranged such that each bendable segment 204A in the first set is spaced apart by a distance F from a distal adjacent bendable segment 204B in the second set on a distal side of the respective bendable segment 204A and by a distance G from a proximal adjacent bendable segment 204B in the second set on a proximal side of the respective bendable segment 204A. In some examples, the distances F and G may be equal or approximately equal as illustrated in Fig. 2a, i.e. the bendable segments 204A, 204B may be arranged equidistantly. In other examples, the distances F and G may be different and the bendable segments 204A, 204B may for example be arranged in pairs as detailed below with reference to Figs. 3b, 8b and 8c.

Fig. 2b shows an enlarged view (not to scale) of a bendable segment 204A of the tube 200 corresponding to the dotted rectangle in Fig. 2a according to an example of the present disclosure. The central connecting portions 208 each have a length A1 along the circumferential direction ϕ and the proximal and distal connecting portions 212A, 212B each have a length A2 along the circumferential direction. The length A1 may for example be between 3% and 10%, in some examples between 4% and 8% of the circumference of the tube 200. The length A2 may be smaller than the length A1 and may for example be between 2% and 5% of the circumference of the tube 200.

In the example of Fig. 2b, the central openings 206 have a rectangular shape with a uniform or substantially uniform width C along the longitudinal direction X, wherein corners of the central openings 206 may be rounded. The width C may for example be between 1% and 5%, in some examples between 2% and 3% of the circumference of the tube 200. In some examples, the width C is between 0.05 mm and 1 mm, in one example between 0.1 mm and 0.5 mm and in one example between 0.2 mm and 0.3 mm.

The proximal and distal openings 210A, 210B are narrow slits having a large aspect ratio. For example, a length of each of the proximal and distal openings 210A, 210B in the circumferential direction maybe between 50 and 200 times, in one example between 100 and 200 times a width of the respective opening in the longitudinal direction. The width of each of the proximal and distal openings 210A, 210B may be less than 20%, in some examples less than 10% of the width C and may for example be between 0.01 mm and 0.05 mm. A length of the central openings 206 in the circumferential direction may for example be between 5 and 15 times, in one example between 8 and 12 times the width C.

The length of the proximal and distal openings 210A, 210B is substantially larger than the length A1 of the central connecting portions 208, for example between three times and ten times as large as the length A1. Accordingly, the proximal and distal openings 210A, 210B overlap with the central openings 206 along the circumferential direction, forming narrow spring segments 214 therebetween. Each of the spring segments 214 has a width D along the longitudinal direction corresponding to the distance between the central openings 206 and the proximal and distal openings 210A, 210B, respectively. The width D is chosen to be sufficiently small such that the spring segments 214 may be deformed (e.g. elongated and/or bent) when the tube is bent, which may be associated with an opening or closing of the respective openings along the longitudinal direction. The width D may for example be smaller than the width C of the central openings 206, for example between 30% and 90% of the width C. In some examples, the width D is between 0.05 mm and 0.5 mm, in one example between 0.1 mm and 0.25 mm.

Fig. 3a shows an enlarged view of a bendable segment 204A/B in accordance with another example of the present disclosure, which may for example also be used in a tube such as the tube 200 of Fig. 2a, either in addition to or instead of the bendable segment 204A of Fig. 2b.

The bendable segment 204A/b of Fig. 3a differs from the one of Fig. 2b in that the central openings 206 do not have a uniform width in the longitudinal direction X, but have a "butterfly-like" shape with a constricted central portion 206C being arranged between a pair of intermediate portions 206B and a pair of end portions 206A. A width C2 of the constricted central portion 206A is smaller than a width C1 of the end portions 206A and smaller than a width C3 of the intermediate portions 206B. In the example of Fig. 3a, the width C2 is between 40% and 60%, e.g. 50% of the width Ci. The width C1 may be slightly smaller than the width C3 and may for example be between 80% and 95% of C₃. The width C3 may be the maximum width of the central opening 206. The end portions 206A of the central opening 206 may include a rounded corner portion with a radius of curvature B, which may for example be between 0.02 mm and 0.3 mm, in one example between 0.05 mm and 0.1 mm. The rounded corner portions may reduce bending and torsional stresses in the vicinity of the end portions 206A in some examples. The width C1 of the end portions 206A may for example be measured at an innermost edge of the rounded corner portions. In some examples, the central opening 206 may be mirror-symmetric with respect to a plane parallel to the longitudinal direction X and extending through the center of the central opening 206.

In the example of Fig. 2b, the proximal and distal openings 210A are curved. A center portion of the proximal openings 210A adjacent to the central connecting portions 208 is closer to the proximal end of the tube than end portions of the proximal openings 210A adjacent to the proximal connecting portions 212A. A center portion of the distal openings 210B adjacent to the central connecting portions 208 is closer to the distal end of the tube than end portions of the distal openings 210B adjacent to the distal connecting portions 212B. In some examples, the proximal openings 210A and/or the distal openings 210B may each be mirror-symmetric with respect to a plane parallel to the longitudinal direction X and extending through the center of the respective opening. Additionally or alternatively, the proximal openings 210A may be mirror-symmetric to the distal openings 210B with respect to a plane perpendicular to the longitudinal direction.

The central opening 206 and the proximal and distal openings 210A, 210B are shaped such that a width of the spring segments 214 formed therebetween varies along their length. A width D3 of the spring segments 214 at their center is smaller than the width D1 at their end adjacent to the proximal/distal connecting portions 212A, 212B and smaller than the width D2 at their end adjacent to the central connecting portion 208. The width D3 may for example be between 70% and 85% of the width D1 and/or of the width D2. In some examples, the width D1 may be smaller than the width D2, for example between 80% and 90% of D2. A length E of the spring segments 214 along the circumferential direction may for example be between 10% and 16%, in one example between 12% and 15% of the circumference of the tube.

Fig. 3b depicts two adjacent bendable segments 204A, 204B as in Fig. 3a, wherein the proximal bendable segment 204A is rotated (or displaced) relative to the distal bendable segment 204B similar as described above for the tube 200 of Fig. 2a. The central connecting portions 208 of the proximal bendable segment 204A are displaced by a distance/displacement H relative to the central connecting portions 208 of the distal bendable segment 204A. The distance H may for example correspond to half the distance/displacement L between adjacent central connecting portions 208 of one of the bendable segments 204A, 204B, cf. Fig. 2a, and may e.g. be 1/6 of the circumference of the tube (corresponding to a rotation by 60°).

The bendable segments 204A, 204B are arranged as a pair of bendable segments, for example such that the distance F between the two bendable segments 204A, 204B of the pair is much smaller than a distance G between the pair and other bendable segments, e.g. as shown in Figs. 8b and 8c. In the example of Fig. 3b, the distance F between the distal openings 210B of the proximal bendable segment 204A and the proximal openings 210A of the distal bendable segment 204B is similar to (e.g. between 80% and 120% of) the distance D between the central openings 206 and the proximal and distal openings 210A, 210B, respectively, such that an additional set of spring segments 216 is formed between the pair of bendable segments.

Similar to the spring segments 214 of Fig. 3a, the spring segments 216 have a smaller width D6 at their center than at their end adjacent to the proximal connecting portion 212A of the distal bendable segment 204B (width D4) and than at their end adjacent to the distal connecting portion 212B of the proximal bendable segment 204A (width D5). The ratios of the widths D4-D6 may for example be within the ranges given above for the ratios of the widths D1-D3. In some examples, the width D4 may be similar to (e.g. between 80% and 120% of) the width Di, the width D5 may be similar to (e.g. between 80% and 120% of) the width D2 and the width D6 maybe similar (e.g. between 80% and 120% of) the width D3.

Fig. 4 depicts a perspective view of a tube 200 according to an example of the present disclosure, wherein the tube 200 is cut perpendicular to the longitudinal X direction for illustration purposes. The tube 200 includes a plurality of bendable segments 204A, 204B with constricted central portions similar to the bendable segment 204A/B of Fig. 3a, wherein two of the bendable segments 204A, 204B are visible in Fig. 4. The bendable segments 204A, 204B are rotated with respect to each other along the circumferential direction similar as described above with reference to Figs. 2a and 3b. In contrast to Fig. 3b, the bendable segments 204A, 204B are arranged spaced apart from each other such that the distance between the pairs is much larger than the distance between central openings 206 and the proximal and distal openings, respectively, of one of the bendable segments 204A, 204B. In this case, no spring segments may be formed between the bendable segments 204A, 204B since the width of the wall portion between the bendable segments 204A, 204B may be too large to permit a significant deformation of said wall portion.

Figs. 5a and 5b depict further examples for bendable segments 204A, 204B in accordance with the present disclosure, wherein the bendable segments 204A, 204B of Figs. 5a and 5b differ from the bendable segments of Figs. 3a, 3b in the shape of the central openings 206 and/or of the proximal and distal openings 206.

In the example of Fig. 5a, the central openings 206 have a "snowboard-like" shape with a constricted central portion 206C arranged between a pair of intermediate portions 206B and a pair of end portions 206A. A width C2 of the constricted central portion 206C is smaller than a width C1 of the end portions 206A and a width C3 of the intermediate portions, e.g. similar as described above for Fig. 3a. In contrast to the example of Fig. 3a, the width C1 may be substantially equal to or slightly larger than the width C3. The width C1 may for example be between 95% and 120%, in one example between 100% and 110% of C3.

In the example of Fig. 5b, the central openings 206 have a "double diamond-like" shape with a pair widened intermediate portions 206B each being arranged between a constricted central portion 206C and a respective tapered end portion 206A. A width C2 of the constricted central portion 206C may for example be between 60% and 70% of a width C3 of the widened intermediate portions 206B. A width C1 of the tapered end portions is substantially equal to or smaller than the width C2. The width C1 may for example be between 40% and 70% of the width C2. In one example, a ratio of the width C2 to the width C3 is similar to (e.g. between 80% and 120% of) a ratio of the width C1 to the width C2.

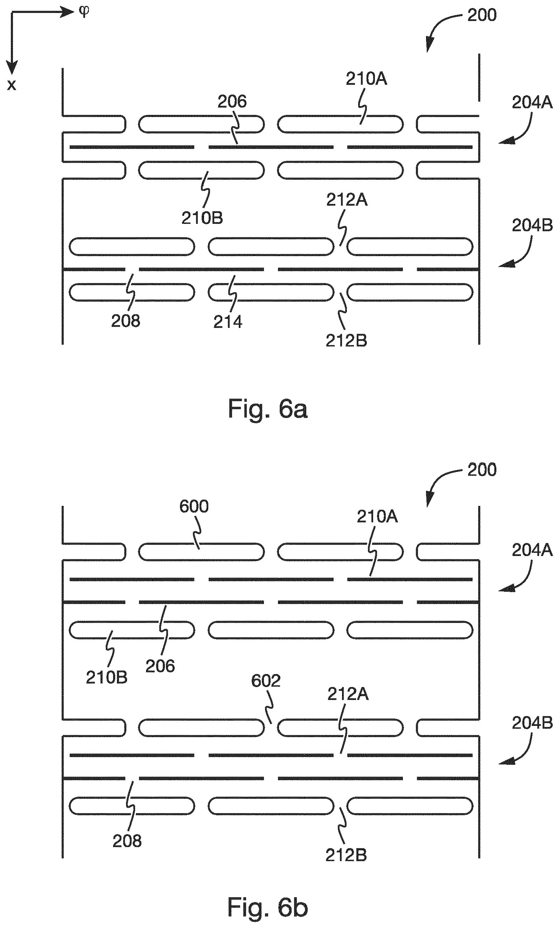

Figs. 6a and 6b depict further examples of tubes 200 including a plurality of bendable segments 204A, 204B according to examples of the present disclosure, wherein the tubes 200 are shown cut open along the longitudinal X direction with their walls 200 rolled out as in Fig. 2a.

The tube 200 of Fig. 6a is similar to the tube of Fig. 2a, except that in this example the central openings 206 are narrow slits similar to the proximal and distal openings of Fig. 2a, whereas the proximal and distal openings 210A, 210B are wider openings similar to the central openings of Fig. 2a. A width of the proximal and distal openings 210A, 210B along the longitudinal direction may for example be at least 5 times, in some examples at least 10 times as large as a width of the central openings 206.

In the example of Fig. 6b, each of the bendable segments 204A, 204B includes four sets of openings, namely two inner sets of openings and two outer sets of openings arranged on the proximal and distal side, respectively, of the two inner sets of openings. Each set of openings is rotated along the circumferential direction such that the openings in the respective set are adjacent to (e.g. aligned with) the connecting portions between the openings of the adjacent sets. In the example of Fig. 6b, the openings in the inner two sets are narrow slits having a much smaller width along the longitudinal direction than the openings in the two outer sets. Bendable segments as in Fig. 6b may for example provide improved torque transfer at a given rigidity/flexibility of the tube 200. Bendable segments as in Fig. 6b may for example be obtained by providing three fourth openings 600 on a proximal side of the proximal openings 210A, wherein the fourth openings 600 are each arranged adjacent to a respective proximal connecting portion 212A and separated from each other by fourth connecting portions 602. Alternatively, the fourth openings 600 may also be provided on a distal side of the distal openings 210B adjacent to a respective one of the distal connecting portions 212B.

Fig. 7 shows a tube 700 for use in a surgical device such as the endoscope 100 of Fig. 1 according to another example of the present disclosure. The tube 700 extends along a longitudinal direction X from a proximal end 700A, which may e.g. be connected or attached to the connector 104 of the endoscope 100, to a distal end 700B. The tube 700 includes five sections 700-I, 700-II, 700-III, 700-IV and 700-V arranged between the proximal and distal ends 700A, 700B, wherein each of the sections 700-I to 700-V may exhibit a different degree of rigidity/flexibility.

In the example of Fig. 7, the tube 700 includes a rigid section 700-I arranged at the proximal end 700A. The rigid section 700-I may not include any bendable segments, e.g. to ensure a robust connection to the connector 104. A length of the rigid section 700-I along the longitudinal direction may for example be between 1% and 10%, in some examples between 2% and 6%, e.g. 4%, of a total length of the tube 700. In one example, the total length of the tube 700 is between 50 cm and 100 cm and the length of the rigid section 700-I is between 1 cm and 5 cm.

The tube 700 further includes three passive bending sections, namely a proximal passive bending section 700-II, a central passive bending section 700-III and a distal passive bending section 700-IV, arranged between the rigid section 700-I and the distal end 700B. The sections 700-II to 700-IV may each exhibit different degrees of rigidity/flexibility, e.g. such that the rigidity increases successively from the section 700-II to the section 700-IV. Put differently, the section 700-II may be more rigid (less flexible) than the section 700-III on the distal side of section 700-II and the section 700-III may be more rigid (less flexible) than the section 700-IV on the distal side of section 700-III. A length of the section 700-II along the longitudinal direction may for example be between 20% and 70%, in some examples between 30% and 50%, e.g. 40%, of the total length of the tube 700. A length of the section 700-III along the longitudinal direction may for example be between 20% and 70%, in some examples between 30% and 50%, e.g. 40% of the total length of the tube 700. In some examples, the length of section 700-III may be similar to (e.g. between 80% and 120% of) the length of section 700-II. A length of the section 700-IV along the longitudinal direction may be substantially smaller than the lengths of sections 700-II, 700-III. The length of section 700-IV may for example be between 1% and 10%, in some examples between 2% and 5%, e.g. 3%, of the total length of the tube 700. In one example, the length of section 700-II is between 20 cm and 40 cm, the length of section 700-III is between 20 cm and 40 cm and the length of section 700-IV is between 1 cm and 4 cm.

A portion of the distal passive bending section 700-IV is depicted in Fig. 8a, a portion of the central passive bending section 700-III in Fig. 8b and a portion of the proximal passive bending section 700-II in Fig. 8c.

Each of the passive bending sections 700-II to 700-IV includes a plurality of bendable segments 204A, 204B arranged along the length of the respective section. To achieve different degrees of rigidity, a density of bendable segments (e.g. a number of bendable segments 204A, 204B per unit length of the tube 700) varies between the sections 700-II to 700-IV with the density of bendable segments in the distal passive bending section 700-IV being higher than in the central passive bending section 700-III and the density of bendable segments in the central passive bending section 700-III being higher than in the proximal passive bending section 700-II.

In the distal passive bending section 700-IV, the bendable segments 204A, 204B are arranged as a contiguous group of bendable segments, wherein both the distance F between a bendable segment 204A and a distal adjacent bendable segment 204B and the distance G between the bendable segment 204A and a proximal adjacent bendable segment 204B (cf. Fig. 2a) is similar to the distance D between the central openings 206 and the proximal and distal openings 210A, 210B within the bendable segments 204A, 204B. In this way, a contiguous arrangement of spring segments may be formed and a high flexibility may be achieved in section 700-IV. The distal passive bending section 700-IV may for example include between 5 and 20, in one example between 10 and 15 bendable segments.

In the central passive bending section 700-III and the proximal passive bending section 700-II, the bendable segments 204A, 204B are arranged in pairs. The distance F between a bendable segment 204A and a distal adjacent bendable segment 204B is similar to the distance D between the central openings 206 and the proximal and distal openings 210A, 210B, respectively, within the bendable segments 204A, 204B to form an additional set of spring segments between the pair of bendable segments 204A. The distance G between the bendable segment 204A and a proximal adjacent bendable segment 204B, on the other hand, is much larger than the distance F such that the density of bendable segments is smaller than in the distal passive bending section 700-IV and no additional spring segments are formed between the pairs. For example, the distance G in sections 700-II, 700-III may be between 5 and 10 times, in one example between 8 and 12 times the distance F in the respective section. To achieve a higher flexibility in the central passive bending section 700-III, the distance G may be smaller in the central passive bending section 700-III than in the proximal passive bending section 700-II in some examples. The distance G in section 700-II may for example be between 100% and 150%, in one example between 105% and 120% of the distance G in section 700-III. Each of the sections 700-II, 700-III may for example include between 50 and 150, in one example between 75 and 100 bendable segments.

In addition to or instead of the density, the sections 700-II to 700-IV may also differ from each other in one or more parameters characterizing an arrangement and/or a shape of the openings in the bendable segments and/or in the pairs of bendable segments to achieve different degrees of rigidity. For example, the length A1 of the central connecting portions 208 may be larger in section 700-III than in section 700-IV and/or may be larger in section 700-II than in section 700-III. The length A2 of the proximal and/or distal connecting portions 212A, 212B may be larger in section 700-III than in section 700-IV and/or may be larger in section 700-II than in section 700-III. The distance D between the central openings 206 and the proximal and distal openings 210A, 210B, respectively, (corresponding to the width of the spring segments 214) may be larger in section 700-III than in section 700-IV and/or may be larger in section 700-II than in section 700-III. The distance F between the bendable segments 204A, 204B of a pair of bendable segments (corresponding to the width of the spring segments 216) may be larger in section 700-III than in section 700-IV and/or may be larger in section 700-II than in section 700-III.

In other examples, the tube 700 may include more or less passive bending sections than in the example of Fig. 7, e.g. only two passive bending sections or four passive bending sections. For example, the proximal and central passive bending sections 700-II, 700-III may instead by embodied as a single passive bending section having a uniform density of bendable segments.

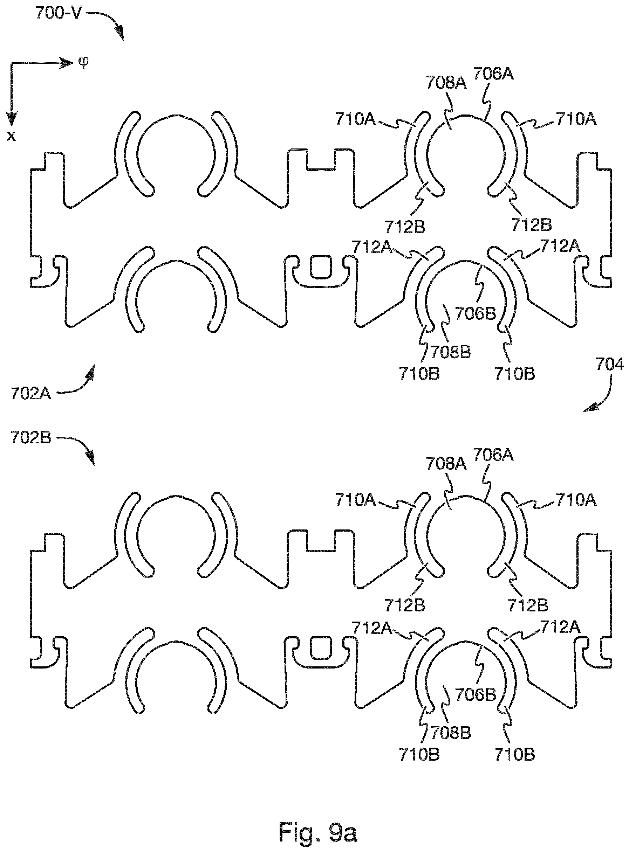

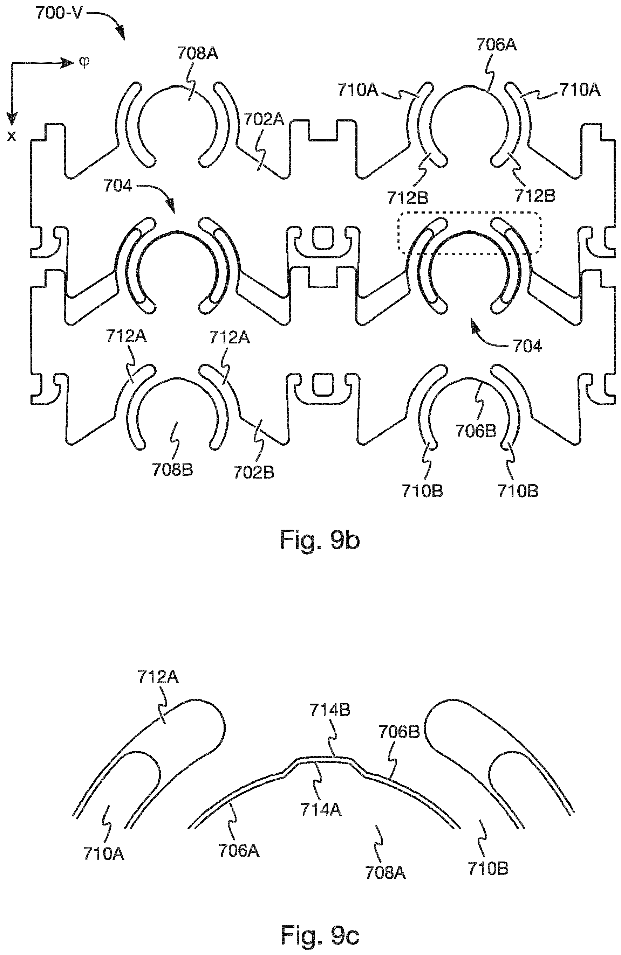

The tube 700 also includes an active bending section 700-V, a portion of which is depicted in Figs. 9a and 9b. The active bending section 700-V is arranged between the distal passive bending section 700-IV and the distal end 700B of the tube 700. A length of the active bending section 700-V may for example be between 4% and 10%, in some examples between 6% and 8% of the total length of the tube 700, e.g. between 2 cm and 5 cm in one example. The active bending section 700-V includes a plurality of annular or tubular elements 702A, 702B that are pivotably connected to each other by a plurality of joints 704, thereby forming a linear chain or sequence of elements that can be articulated with respect to each other. Each of the joints 704 connects two adjacent elements, namely a respective proximal element 702A and a respective distal element 702B, to each other. In Fig. 9a, a joint 704 is shown with the proximal and distal elements 702A, 702B associated with this joint 704 taken apart for illustration purposes, whereas Fig. 9b depicts the joint 704 with the proximal and distal elements 702A, 702B being arranged as in the active bending section 700-V during normal use of the tube 700 of Fig. 7. In the example of Figs. 9a, 9b, adjacent elements 702A, 702B are connected to each other by a pair of joints 704 arranged on opposite sides of the tube 700 (e.g. rotated by 180° along the circumferential direction). Fig. 9c depicts a close-up view of one of the joints 704 corresponding to the dashed rectangle in Fig. 9b. The joints 704 may for example be actuated (e.g. articulated) by one or more control wires as commonly known in the art. For example, the flaps arranged halfway between the joints 704 on the elements 702A, 702B may be bent inwards into the interior of the tube 700 and the openings or cut-outs in the flaps may provide a guiding channel in which a respective control wire can be arranged.

Each of the joints 704 includes a distal contact surface 706B on a respective proximal element 702A (i.e. the proximal element 702A associated with the respective joint) and a proximal contact surface 706A on a respective distal element 702B (i.e. the distal element 702B associated with the respective joint). In the example of Figs. 9a, 9b, the proximal contact surface 706A is formed by a surface of a joint head 708A (e.g. a circular joint head having the shape of a circular segment as shown in Figs. 9a, 9b) protruding from the distal element 702B of the joint 704, e.g. by a side face of the joint head 708A extending in a radial direction corresponding to the direction of view of Figs. 9a, 9b. The distal contact surface 706B is formed by an edge or rim of a joint socket 708B (e.g. circular joint socket having the shape of a circular segment as shown in Figs. 9a, 9b) in the proximal element 702A of the joint 704, e.g. by a side face or wall of the joint socket 708B extending in the radial direction. The joint socket 708B is configured to receive the joint head 708A such that the proximal and distal contact surfaces 706A, 706B slidably engage with each other and the joint head 708A can be rotated within the joint socket 708B.

Each of the joints 704 further includes a pair of arc-shaped proximal brackets 710A protruding from the respective distal element 702B and a pair of corresponding recesses 712A in the respective proximal element 702A, each of which is configured to slidably receive a respective one of the proximal brackets 710A. Each of the joints 704 also includes a pair of arc-shaped distal brackets 710B protruding from the respective proximal element 702A and a pair of corresponding recesses 712B in the respective distal element 702B, each of which is configured to receive a respective one of the distal brackets 710A. In this way, a double joint structure is formed in each of the joints 704, which may increase the robustness of the joints 704 with regard to bending and/or torsional forces.

In the example of Figs. 9a, 9b, the joints 704 on the elements 702A, 702B are aligned with each other along the circumferential direction and arranged on opposite sides of the tube 700, e.g. to allow articulation or bending of the section 700-V in a single plane/direction. In other examples, joints on opposite sides of the elements 702A, 702B may be rotated with respect to each other by 90° along the circumferential direction, e.g. such that the joint heads 708A, the proximal brackets 710A and the proximal recesses 712B on the proximal side of the respective element are arranged between the joint sockets 708B, the distal brackets 710B and the distal recesses 712A on the distal side of the respective element. This may allow for articulation or bending of the section 700-V in two orthogonal planes/directions.

Each of the joints 704 includes a reference protrusion 714A arranged on the proximal contact surface 706A, e.g. at a tip of the joint head 708A, and a corresponding reference notch 714B arranged on the distal contact surface 706B that is configured to receive the reference protrusion 714A as illustrated in Fig. 9c. The reference protrusion 714A and the reference notch 714B together define a reference position for the respective joint 704, which in the example of Figs. 9a-c corresponds to a straight configuration in which the respective proximal and distal elements 702A, 702B are parallel to each other as illustrated in Figs. 9a, 9b (e.g. corresponding to an articulation angle of o°).

The reference protrusion 714A and the reference notch 714B are configured such that when the respective joint is moved out of the reference position, e.g. to a second position corresponding to a bent configuration in which the respective proximal and distal elements 702A, 702B are tilted with respect to each other (e.g. corresponding to a non-zero articulation angle), the length of the tube 700 increases. For example, a tip of the reference protrusion 714A may come in contact with and press against a portion of the distal contact surface 706B adjacent to the reference notch 714B when the joint 704 is rotated away from the reference position, thereby increasing a spacing between the respective proximal and distal elements 702A, 702B and thus a length of the tube 700. As a result, a force that is required for pivoting the proximal and distal elements with respect to each other is higher when the joint is in the reference position (articulation angle of 0°, reference protrusion 714A arranged in reference notch 714B) than when the joint is in the second position (non-zero articulation angle, reference protrusion 714A arranged outside of the reference notch 714B). This may for example ensure that the joints 704 are preferably arranged in the reference configuration and may e.g. prevent the elements of the section 700-V arranging in a zig-zag pattern when the section 700-V is not under tension.

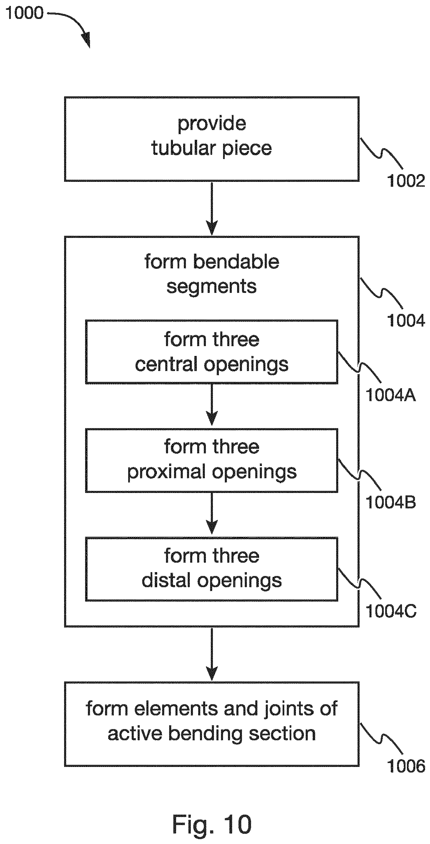

Fig. 10 shows a flow chart of a method 1000 of manufacturing a tube for use in a surgical device that is to be inserted into the human or animal body at least in part according to an example of the present disclosure. The method 1000 may for example be used to manufacture the tube 700 of Fig. 7, which is used as a non-limiting example for illustration purposes in the following. This is, however, not intended to be limiting in any way and the method 1000 may also be used to manufacture other tubes for use in surgical devices, in particular other tubes according to any of the examples of the present disclosure described herein, for example the tube 200 of Fig. 2a. Furthermore, the method 1000 is not limited to the order of execution shown in the flowchart of Fig. 10. As far as technically feasible, the method 1000 may be executed in an arbitrary order and parts thereof may be executed simultaneously at least in part, for example some or all of steps 1004A-C and 1006.

In step 1002, a tubular piece is provided. The tubular piece may for example be a cylindrical tube having a length and a diameter that maybe chosen depending on the type of surgical device and application that the tube 700 is to be used in. The tubular piece may for example have a length between 20 cm and 200 cm, in some examples between 50 cm and 150 cm. An outer diameter of the tubular piece may for example be between 1 mm and 20 mm, in some examples between 2 mm and 10 mm, in one example between 2.5 mm and 5.5 mm. The tubular piece may include (i.e. including, but not limited to) a rigid or semi-rigid material, in particular a metal such as stainless steel. In one example, the tubular piece may consist of (i.e. including nothing else but) a rigid or semi-rigid material, in particular a metal such as stainless steel. A wall thickness of the tubular piece may be chosen depending on the material used and the intended application and may for example be between 0.05 mm and 1 mm, in some examples between 0.1 mm and 0.3 mm. The tubular piece may be formed as a single continuous piece of material.

In step 1004, a plurality of bendable segments 204A, 204B are formed in the tubular piece. Each of the bendable segments 204A, 204B is formed by forming, in the wall of the tubular piece, three central openings 206 (step 1004A), three first openings 210A on a first side of the central openings 206 (step 1004B) and three second openings 210B on a second side of the central openings 206 opposite to the first side (step 1004C).

The central openings 206 are formed displaced from each other along a circumference of the tubular piece by between 29% and 38%, in one example by between 32.8% and 33.9% (e.g. 1/3) of the circumference of the tubular piece in the respective bendable segment. The central openings 206 are formed such that central connecting portions 208 of the wall of the tube 700 remain between the central openings 206. The central openings 206 may for example be formed with a shape, a size and/or an arrangement as described above with reference to Figs. 2a-8c.

The first openings 210A are formed adjacent to a respective one of the central connecting portions 208. The first openings 210A may for example be formed on a proximal side of the central openings 206 and may thus correspond to the proximal openings in the examples described above. The first openings 210A may for example be formed with a shape, a size and/or an arrangement as described above for the proximal openings of Figs. 2a-8c.

The second openings 210B are also formed adjacent to a respective one of the central connecting portions 208, opposing the first openings 210B. The second openings 210B may for example be formed on a distal side of the central openings 206 and may thus correspond to the distal openings in the examples described above. The second openings 210B may for example be formed with a shape, a size and/or an arrangement as described above for the distal openings of Figs. 2a-8c.

The openings 206, 210A, 210B may be formed successively one after the other or may be formed simultaneously at least in part. Each of the openings 206, 210A, 210B may be formed by cutting the wall of the tubular piece, for example by laser cutting. Some or all of the openings 206, 210A, 210B may be formed as slits, e.g. the first/proximal and second/distal openings 210A, 210B as in the example of Fig. 2a or the central openings 206 as in the example of Fig. 6a. In some examples, a width of the slits may correspond to a cutting width of the laser cutting, which may for example be between 10 µm and 50 µm, in one example between 20 µm and 40 µm. The cutting width of the laser cutting may for example be determined by a focal diameter or width of a laser beam used to perform the cutting.

The bendable segments 206A, 206B may be formed in one or more sections of the tubular piece, for example as described above with reference to Fig. 7. In some examples, step 1004 may include forming additional openings in some or all of the bendable segment, for example a fourth set of openings as in the example of Fig. 6b.

The method 1000 may further include forming an active bending section such as section 700-V of the tube 700 that includes a plurality of annular or tubular elements 702A, 702B that are pivotably connected to each other by a plurality of joints 704. The elements 702A, 702B and joints 704 of the active bending section 700-V may in particular also be formed of the same tubular piece that the bendable segments 204A, 204B are formed in. For example, the elements 702A, 702B may be cut out of the tubular piece, e.g. by laser cutting. The elements 702A, 702B may be formed having a shape as described above with reference to Figs. 9a-c, e.g. by cutting along an outer circumference or edge of the respective element 702A, 702B.

The examples of the present disclosure disclosed herein only constitute specific examples for illustration purposes. The present invention can be implemented in various ways and with many modifications without altering the underlying basic properties. Therefore, the present invention is only defined by the claims as stated below.

### LIST OF REFERENCE SIGNS

- 100 -: surgical device/endoscope
- 102 -: interface/control portion
- 104 -: connector
- 106 -: port

- 200 -: tube
- 200A -: proximal end
- 200B -: distal end
- 202 -: wall
- 204A -: bendable segment of first type/set
- 204B -: bendable segment of second type/set
- 206 -: central opening
- 206A -: end portion of central opening 206
- 206B -: intermediate portion of central opening 206
- 206C -: central portion of central opening 206
- 208 -: central connecting portion
- 210A -: first/proximal opening
- 210B -: second/distal opening
- 212A -: proximal connecting portion
- 212B -: distal connecting portion
- 214, 216: - spring segment

- 600 -: fourth opening
- 602 -: fourth connecting portion

- 700 -: tube
- 700A -: proximal end
- 700B -: distal end
- 700-I -: rigid section
- 700-II -: proximal passive bending section
- 700-III -: central passive bending section
- 700-IV -: distal passive bending section
- 700-V -: active bending section
- 702A -: proximal element
- 702B -: distal element
- 704 -: joint

- 706A -: proximal contact surface
- 706B -: distal contact surface
- 708A -: joint head
- 708B -: joint socket
- 710A -: proximal bracket
- 710B -: distal bracket
- 712A -: recess for proximal bracket 710A
- 712B -: recess for distal bracket 710B
- 714A -: reference protrusion
- 714B -: reference notch

- 1000 -: method of manufacturing a tube
- 1002 -: step of providing a tubular piece
- 1004 -: step of forming bendable segments
- 1004A -: step of forming central openings
- 1004B -: step of forming proximal openings
- 1004C -: step of forming distal openings
- 1006 -: step of forming active bending section

- X -: longitudinal direction
- ϕ -: circumferential direction

- A1 -: length of central connecting portion
- A2 -: length of proximal/distal connecting portion
- B -: bending radius of end portion of central opening
- C, C1, C2, C3 -: width of central opening
- D -: distance between central and proximal/distal opening
- D, D1, D2, D3 -: width of spring segment 214
- D4, D5, D6, F -: width of spring segment 216
- E -: length of spring segment 214, 216
- F, G -: distance between adjacent bendable segments
- L -: displacement of adjacent central connecting portions

## Claims

1. A tube (200, 700) for use in a surgical device (100) that is to be inserted into the human or animal body at least in part, the tube (200, 700) extending from a proximal end (200A, 700A) to a distal end (200B, 700B) of the tube (200, 700), wherein the tube (200, 700) comprises a plurality of bendable segments (204A, 204B), each of the bendable segments (204A, 204B) comprising:
three central openings (206) in a wall (202) of the tube (200), the central openings (206) being arranged along a circumference of the tube (200) and separated from each other by central connecting portions (208) of the wall (200), wherein the central connecting portions (208) are displaced from each other by between 105° and 135° along the circumference of the tube (200);
three proximal openings (210A) in the wall (202) arranged on a proximal side of the central openings (206) with each of the proximal openings (210A) being adjacent to a respective one of the central connecting portions (208); and
three distal openings (210B) in the wall (202) arranged on a distal side of the central openings (206) with each of the distal openings (210B) being adjacent to a respective one of the central connecting portions (208).

2. The tube (200, 700) of claim 1, wherein at least some of the bendable segments (204A, 204B) are arranged in pairs along a longitudinal direction (X) of the tube (200,700) with a distance (F) between the bendable segments (204A, 204B) of each pair being less than 50%, preferably less than 25% of a distance (G) between the respective pair and an adjacent pair.

3. The tube (200, 700) of claim 1 or 2, wherein a distance (F) between the distal openings (210B) of a proximal bendable segment (204A) and the proximal openings (210A) of a distal bendable segment (204B) of two adjacent bendable segments of the plurality of bendable segments (204A, 204B) is between 50% and 200%, preferably between 80% and 120% of a distance (D) between the proximal openings (210A) and the central openings (206) of the distal bendable segment (204B) and/or between 50% and 200%, preferably between 80% and 120% of a distance (D) between the central openings (206) and the distal openings (212B) of the proximal bendable segment (204A), in particular wherein:
the proximal openings (210A) of the distal bendable segment (204B) and the distal openings (210B) of the proximal bendable segment (204A) overlap along a circumferential direction (ϕ) of the tube (200, 700) forming spring segments (216) therebetween, wherein preferably a width (D6) of one or more of said spring segments (216) along the longitudinal direction (X) at a center of the respective spring segment (216) is smaller than a width (D4, D5) of the respective spring segment (216) at one or both ends of the respective spring segment (216); and/or
at least some of the bendable segments (204A, 204B) are arranged as a contiguous group of bendable segments with the distance (F) between the distal openings (210B) of a proximal bendable segment (204A) and the proximal openings (210A) of a distal bendable segment (204B) of any two adjacent bendable segments in the contiguous group of bendable segments being between 50% and 200% of the distance (D) between the proximal openings (210A) and the central openings (206) of the distal bendable segment (204B) and/or between 50% and 200% of the distance (D) between the central openings (206) and the distal openings (210B) of the proximal bendable segment (204A).

4. The tube (200, 700) of any one of the preceding claims, wherein:
adjacent bendable segments (204A, 204B) of the plurality of bendable segments (204A, 204B) are rotated with respect to each other along the circumferential direction (ϕ) with centers of the central openings (206) of one (204A) of said adjacent bendable segments (204A, 204B) being aligned with the central connecting portions (208) of another one (204B) of said adjacent segments (204A, 204B); and/or
the proximal openings (210A) and/or the distal openings (210B) are slits with a width (C) of the central openings (206) along the longitudinal direction (X) being at least 5 times, preferably at least 10 times as large as a width of the slits.

5. The tube (200, 700) of any one of the preceding claims, wherein the proximal openings (210A) are separated from each other by proximal connecting portions (212A) of the wall (202) and the distal openings (210B) are separated from each other by distal connecting portions (212B) of the wall (202), in particular wherein:
a length (A2) of the proximal connecting portions (212A) and/or a length (A2) of the distal connecting portions (212B) along the circumferential direction (ϕ) is between 25% and 200%, preferably between 30% and 100% of a length (A1) of the central connecting portions (208); and/or
the length (A2) of the proximal connecting portions (212A) and/or the length (A2) of the distal connecting portions (212B) is no more than 10%, preferably no more than 5% of the circumference of the tube (200, 700) in the respective bendable segment (204A, 204B); and/or
some or all of the bendable segments (204A, 204B) each further comprise a fourth set of openings, the fourth set of openings comprising three fourth openings (600) that are arranged on a proximal side of the proximal openings (210A) with each of the fourth openings (600) being adjacent to a respective one of the proximal connecting portions (212A) or arranged on a distal side of the distal openings (210B) with each of the fourth openings (600) being adjacent to a respective one of the distal connecting portions (212B).

6. The tube (200, 700) of any one of the preceding claims, wherein the proximal openings (210A) and/or the distal openings (210B) of some or all of the bendable segments (204A, 204B) overlap with the central openings (206) of the respective bendable segment (204A, 204B) along the circumferential direction (ϕ) forming spring segments (214) therebetween, wherein a width (D3) of one or more of said spring segments (214) along the longitudinal direction (X) at a center of the respective spring segment (214) is smaller than a width (Di, D2) of the respective spring segment (214) at one or both ends of the respective spring segment (214).

7. The tube (200, 700) of any one of the preceding claims, wherein:
some or all of the central openings (206), some or all of the proximal openings (210A) and/or some or all of the distal openings (210B) each comprise a constricted central portion (206C) at a center of the respective opening (206, 210A, 210B), the constricted central portion (206C) having a width (C2) along the longitudinal direction (X) that is between 20% and 90%, preferably between 40% and 70% of a maximum width of the respective opening (206, 210A, 210B); and/or
a width (C2) of some or all of the central openings (206), a width of some or all of the proximal openings (210A) and/or a width of some or all of the distal openings (210B) along the longitudinal direction (X) at the center of the respective opening (206, 210A, 210B) is between 20% and 90%, preferably between 40% and 70% of a width (C1) of one or both end portions (206A) of the respective opening (206, 210A, 210B); and/or
some or all of the central openings (206), some or all of the proximal openings (210A) and/or some or all of the distal openings (210B) each comprise widened intermediate portions (206B) arranged between the central portion (206C) and a respective end portion (206A) of the respective opening (206, 210A, 210B), a width (C3) of the widened intermediate portions (206B) along the longitudinal direction (X) being larger than the width (C2) of the central portion (206C) and larger than a width (C1) of the end portions (206A), in particular wherein the width (C1) of one or both end portions (206A) of the respective opening (206, 210A, 210B) is between 40% and 90% of the width (C2) of the central portion (206C).

8. The tube (200, 700) of any one of the preceding claims, the tube (200, 700) comprising a first section (700-II) and a second section (700-III, 700-IV), wherein a density of bendable segments in the first section (700-II) is different from a density of bendable segments in the second section (700-III, 700-IV), in particular wherein:
the bendable segments (204A, 204B) in the second section (700-IV) are arranged as a contiguous group of bendable segments with a distance (F) between the distal openings (210B) of a proximal bendable segment (204A) and the proximal openings (210A) of a distal bendable segment (204B) of any two adjacent bendable segments in the contiguous group of bendable segments being between 50% and 200% of a distance (D) between the proximal openings (210A) and the central openings (206) of the distal bendable segment (204B) and/or between 50% and 200% of a distance (D) between the central openings (206) and the distal openings (210B) of the proximal bendable segment (204A); and/or
the bendable segments (204A, 204B) in the first section (700-II) are arranged in pairs along the longitudinal direction (X) with the distance (F) between the bendable segments (204A, 204B) of each pair being less than 50%, preferably less than 25% of the distance (G) between the respective pair and an adjacent pair, in particular wherein the bendable segments (204A, 204B) in the second section (700-III) are arranged in pairs along the longitudinal direction (X) with the distance (F) between the bendable segments (204A, 204B) of each pair being less than 50%, preferably less than 25% of the distance (G) between the respective pair and an adjacent pair, and/or the tube (200, 700) comprises one or more additional sections (700-111) in which bendable segments (204A, 204B) are arranged in pairs along the longitudinal direction (X) with the distance (F) between the bendable segments (204A, 204B) of each pair being less than 50%, preferably less than 25% of the distance (G) between the respective pair and an adjacent pair, wherein a density of bendable segments in each of said one or more additional sections (700-111) is different from at least a density of bendable segments in the first section (700-II) and/or in the second section (700-IV).

9. The tube (200, 700) of claim 8, wherein the bendable segments (204A, 204B) in the first section (700-II) differ from the bendable segments (204A, 204B) in the second section (700-III, 700-IV) and/or from the bendable segments (204A, 204B) in some or all of the one or more additional sections (700-111) in one or more of:
the distance (D) between the proximal openings (210A) and the central openings (206) and/or the distance (D) between the central openings (206) and the distal openings (210A);
the distance (F) between bendable segments (204A, 204B) within a pair of bendable segments;
the length (A2) of the proximal connecting portions (212A) between the proximal openings (210A) and/or the length (A2) of the distal connecting portions (212B) between the distal openings (210B); and
the length (A1) of the central connecting portions (208) between the central openings (206).

10. The tube (200, 700) of any one of the preceding claims, further comprising an active bending section (700-V) with a plurality of annular or tubular elements (702A, 702B) that are pivotably connected to each other by a plurality of joints (704).

11. The tube (200, 700) of claim 10, wherein:
some or all of the joints (704) each comprise a distal contact surface (706B) on a proximal element (702A) associated with the respective joint (704) that is configured to slidably engage a proximal contact surface (706A) on a distal element (702B) associated with the respective joint (704), wherein a reference protrusion (714A) is arranged on one of the proximal contact surface (706A) and the distal contact surface (706B) and/or a reference notch (714B) is arranged on another one of the proximal contact surface (706A) and the distal contact surface (706B), the reference protrusion (714A) and/or the reference notch (714B) being arranged such that a force that is required for pivoting the proximal and distal elements (702A, 702B) with respect to each other is higher when the joint (704) is in a reference position than when the joint (704) is in a second position different from the reference position, in particular wherein the reference protrusion (714A) and/or the reference notch (714B) are arranged such that a length of the tube (200, 700) increases when the joint (704) is moved from the reference position to the second position; and/or
the proximal element (702A) and the distal element (702B) are parallel to each other when the respective joint (704) is in the reference position.

12. The tube (200, 700) of claim 10 or 11, wherein some or all of the joints (704) each comprise:
a pair of arc-shaped proximal brackets (710A) on a distal element (702B) associated with the respective joint (704), each of the proximal brackets (710A) extending along a circular arc; and/or
a pair of arc-shape distal brackets (710B) on a proximal element (702A) associated with the respective joint (704), each of the distal brackets (710B) extending along a respective circular arc,
wherein each of the proximal brackets (710A) is configured to slidably engage with a corresponding recess (712A) in the proximal element (702A) and/or with one of the distal brackets (710B) and/or wherein each of the distal brackets (710B) is configured to slidably engage with a corresponding recess (712B) in the distal element (702B) and/or with one of the proximal brackets (710A),
in particular wherein the proximal brackets (710A) or the distal brackets (710B) form a circular joint socket (708B) configured to receive a corresponding joint head (708A) on the distal element (702B) and the proximal element (702A), respectively, wherein the reference protrusion (714B) is arranged on a surface of one of the joint socket (708B) and the joint head (708A) and/or the reference notch (714B) is arranged on a surface of another one of the joint socket (708B) and the joint head (708A).

13. The tube (200, 700) of any one of the preceding claims, wherein the surgical device (100) is an endoscope (100) and the tube (200, 700) is an insertion tube or a hypotube for use in said endoscope (100).

14. A method (1000) of manufacturing a tube (200, 700) for use in a surgical device (100) that is to be inserted into the human or animal body at least in part, wherein the method (1000) comprises providing a tubular piece and forming a plurality of bendable segments (204A, 204B) in the tubular piece, each of the bendable segments (204A, 204B) being formed by:
forming three central openings (206) in a wall (202) of the tubular piece, the central openings (206) being displaced from each other along a circumference of the tubular piece by between 29% and 38% of the circumference of the tubular piece and separated from each other by central connecting portions (208) of the wall (202);
forming three first openings (210A) in the wall (202) on a first side of the central openings (206) with each of the first openings (210A) being formed adjacent to a respective one of the central connecting portions (208); and
forming three second openings (210B) in the wall (202) on a second side of the central openings (206) opposite to the first side, wherein each of the second openings (210B) is formed adjacent to a respective one of the central connecting portions (208).

15. The method (1000) of claim 14, wherein the first openings (210A) and the second openings (210B) are slits formed by laser cutting and a width of the first and second openings (210A, 210B) along a longitudinal direction (X) of the tubular piece corresponds to a cutting width of the laser cutting, the cutting width preferably being between 10 µm and 50 µm.
